# EUROPEAN PATENT APPLICATION

(11) **EP 4 732 864 A1**
(43) Date of publication of application: **29.04.2026**
(21) Application number: 24825015.1
(22) Date of filing: 30.04.2024
(51) Int. Cl.: A61M 1/36, A61B 17/12

(54) **REVASCULARIZATION SYSTEM**

(30) Priority: 21.06.2023 CN 202310748062
(71) Applicant: Shenzhen Wecan Medical Technology Co., Ltd., Shenzhen, Guangdong 518063 (CN)
(72) Inventor: ZHANG, Wayne Wei, Shenzhen, Guangdong 518000 (CN); CHEN, Zhong, Shenzhen, Guangdong 518000 (CN); WU, Liping, Shenzhen, Guangdong 518000 (CN); ZHANG, Xiong, Shenzhen, Guangdong 518000 (CN); LI, Zhen, Shenzhen, Guangdong 518000 (CN)
(74) Representative: Michalski Hüttermann & Partner Patentanwälte mbB
(86) International application number: PCT/CN2024/090920
(87) International publication number: WO 2024/260117

(57) **Abstract**

The present disclosure relates to a revascularization system, comprising an arterial sheath, a bypass system, and a venous sheath which are connected in sequence. The arterial sheath is configured to be connected to the carotid artery, and the venous sheath is configured to be connected to a vein. The arterial sheath comprises a main body and a catheter seat located at the proximal end of the main body, and the proximal end of the catheter seat is communicated with a stent system that is used for loading and delivering a stent to the distal end of the main body. The bypass system comprises an inflow tube, an outflow tube, and a bypass assembly. A tube cavity is arranged in the bypass assembly, a blood filter is arranged in the tube cavity, and the blood in the tube cavity flows from the artery sheath to the venous sheath. The revascularization system of the present disclosure can establish reverse flow of blood from the carotid artery to the femoral vein, preventing blood clots that have detached during surgery from entering the brain.

## Description

### Technical Field

The present disclosure relates to the technical field of medical devices, and more particularly to a revascularization system.

### Background Art

Carotid artery disease typically occurs due to the accumulation of thrombi or plaques along lateral walls of the common carotid artery and the internal carotid artery (arteries responsible for supplying blood from the heart to the brain), which leads to a narrowing of arterial lumen. These accumulations easily detach and form thrombi that travel with the blood flow. Once these thrombi enter the brain via the carotid arteries, they may cause a stroke.

Among surgical treatments for carotid artery stenosis, carotid endarterectomy (CEA) is regarded as the standard procedure. In this surgery, a relatively large incision is made in the neck of a patient, the carotid artery is clamped upstream and downstream of a target site to block blood flow, the carotid artery is then incised to remove the accumulations, and upon completion of removal, the carotid artery is sutured and blood flow is restored; and however, this surgical solution is highly invasive, carries a risk of damaging cranial nerves near the carotid artery, and still presents a considerable postoperative risk of stroke. Carotid artery stenting (CAS) is a minimally invasive surgery for the treatment of carotid artery stenosis. The procedure involves introducing a delivery sheath into the femoral artery via percutaneous puncture, navigating the delivery sheath along the aortic arch into the carotid artery, subsequently deploying a self-expanding stent within the carotid artery to restore normal blood flow of the carotid artery. However, an overall path in CAS is relatively long, and during the deployment of the delivery sheath and the expansion of the self-expanding stent, accumulations on the inner wall of the carotid artery may easily detach. CAS has a significant higher risk of postoperative stroke compared to CEA and presents clear limitations in clinical application.

### Summary of the Disclosure

In view of the foregoing, there is a need to provide a medical device for treating carotid artery stenosis, which minimizes invasiveness and reduces a risk of stroke.

The present disclosure provides a revascularization system, including a first sheath, a bypass system, and a second sheath, which are sequentially connected. The first sheath includes a main body and a catheter seat located at a proximal end of the main body. A proximal end of the catheter seat may be communicated with a stent system. The stent system is configured to load and deliver a stent to a distal end of the main body. The bypass system includes an inflow tube in communication with the first sheath, an outflow tube in communication with the second sheath, and a bypass assembly connected between the inflow tube and the outflow tube. The bypass assembly includes a tube cavity in which a blood filter is arranged, and blood flows from the first sheath to the second sheath via the bypass system.

In one embodiment, the first sheath includes a tubular main body and a balloon located at a distal end of the main body. The balloon is located on a distal side of the first sheath, and an inflation port of the balloon is communicated with the balloon via the catheter seat.

In one embodiment, the main body includes an inflation lumen which is communicated between the balloon and the catheter seat. When inflated, the balloon blocks an outer periphery of the main body and exposes an inner lumen of the main body.

In one embodiment, the main body further includes at least one angiography lumen.

In one embodiment, the bypass system includes a flow controller connected to an end of the inflow tube that is away from the first sheath. The flow controller includes a main body element with a flow channel and a flow control element, and the flow control element is at least partially accommodated in the main body element and movably connected to the flow channel. Under an external force, the flow control element reduces or increases the flow area of the flow channel.

In one embodiment, the bypass system includes a one-way check valve, which is connected between the blood filter and the outflow tube, or connected to the outflow tube.

In one embodiment, the flow channel is provided with an inlet end and an outlet end. The inlet end is close to the inflow tube and the outlet end is far away from the inflow tube. The flow control element is provided with a guiding surface oriented to the inlet end. The guiding surface and an inner surface of the flow channel define a flow-regulating channel that tapers from the inlet end to the outlet end.

In one embodiment, the blood filter includes a filter mesh that is spaced from an inner wall of the tube cavity; or the blood filter further includes a filter cartridge which has a tubular structure with at least one open end; and the filter mesh is arranged inside the filter cartridge and is spaced from an inner wall of the filter cartridge.

In one embodiment, the flow channel includes a connection section, a constricted section, and a main section, which are sequentially connected from the inlet end to the outlet end; and the inner diameter of the constricted section is smaller than that of the connection section and the main section.

In one embodiment, the catheter seat includes at least a first cavity, a second cavity, and a third cavity. The first cavity and the third cavity are located on two sides of the second cavity respectively. A first connection plate is connected with the first cavity and the second cavity, and a second connection plate is connected with the third cavity and the second cavity. Each of the first connection plate and the second connection plate is provided with at least one suture hole.

In one embodiment, the stent includes a plurality of main corrugated rings. Each corrugated ring includes at least two crests and/or troughs of different heights. The main corrugated rings include a first corrugated ring and a second corrugated ring, between which a phase difference is provided, such that a plurality of troughs of the first corrugated ring are aligned with a plurality of crests of the second corrugated ring, and/or a plurality of crests of the first corrugated ring are align to a plurality of troughs of the second corrugated ring.

In one embodiment, the first corrugated ring and the second corrugated ring are distributed in an inclined manner.

In one embodiment, the stent includes a main section and radiopaque sections located on two sides of the main section, and each radiopaque section includes a plurality of radiopaque markers distributed in a circumferential direction.

In one embodiment, in each radiopaque section, a connection section between adjacent corrugated rings extends in an axial direction.

In one embodiment, in the main section, a connection section between adjacent corrugated rings extends substantially in the circumferential direction.

The revascularization system of the present disclosure is capable of establishing a retrograde blood flow pathway from the carotid artery to the femoral vein. Before a stent is deployed at a stenotic site of the carotid artery, the first sheath of the present disclosure is introduced into the common carotid artery, and the second sheath is introduced into the femoral vein. After the revascularization system is established, a pressure difference is formed between a high-pressure arterial blood flow of the brain and a low-pressure venous blood flow of the femoral vein, causing the blood flow in the carotid artery to reverse direction and flow toward the femoral vein through the revascularization system. After the revascularization system is established, procedures such as pre-dilation of the stenotic site of the carotid artery and deployment of a self-expanding stent are performed. As a result, any thrombus detached during surgery is intercepted by the blood filter as it is carried by a retrograde blood flow, thereby preventing thrombus from entering cerebral vessels via anterograde blood flow and reducing the risk of stroke.

### Brief Description of the Drawings

FIG. 1 is a schematic structural diagram of a revascularization system according to one embodiment of the present disclosure;
FIG. 2 is a schematic diagram of a revascularization system according to one embodiment of the present disclosure;
FIG. 3 is a schematic structural diagram of an arterial sheath according to one embodiment of the present disclosure;
FIG. 4 is a schematic structural diagram of an arterial sheath according to another embodiment of the present disclosure;
FIG. 5 is a schematic structural diagram of a balloon portion of an arterial sheath according to one embodiment of the present disclosure;
FIG. 6 is a schematic diagram of a first operating principle of a balloon portion of an arterial sheath according to one embodiment of the present disclosure;
FIG. 7 is a schematic diagram of an operating principle of a balloon portion of an arterial sheath according to one embodiment of the present disclosure;
FIG. 8 is a schematic structural diagram of a balloon portion of an arterial sheath according to another embodiment of the present disclosure;
FIG. 9 is a schematic sectional view of the position A-A of FIG. 8 from left to right;
FIG. 10 is a schematic diagram of a first sectional structure of a balloon portion of an arterial sheath according to yet another embodiment of the present disclosure;
FIG. 11 is a schematic sectional view of the position B-B of FIG. 10 from left to right;
FIG. 12 is a schematic diagram of a second sectional structure of a balloon portion of an arterial sheath according to another embodiment of the present disclosure;
FIG. 13 is a schematic structural diagram of a balloon portion of an arterial sheath according to another embodiment of the present disclosure;
FIG. 14 is a schematic sectional view of the position C-C of FIG. 13 from left to right;
FIG. 15 is a schematic structural diagram of a catheter seat according to one embodiment of the present disclosure;
FIG. 16 is a schematic structural diagram of a variant of a catheter seat according to one embodiment of the present disclosure;
FIG. 17 is a schematic structural diagram of another variant of a catheter seat according to one embodiment of the present disclosure;
FIG. 18 is a schematic structural diagram of a catheter seat according to another embodiment of the present disclosure;
FIG. 19 is a sectional view along the position A-A in FIG. 2;
FIG. 20 is a diagram of an exploded view of a flow controller according to one embodiment of the present disclosure;
FIG. 21 is a sectional view of a flow controller in a partially open state according to one embodiment of the present disclosure;
FIG. 22 is a sectional view of a flow controller in a closed state according to one embodiment of the present disclosure;
FIG. 23 is an exploded view of a flow controller according to one embodiment of the present disclosure;
FIG. 24 is a sectional view of a flow controller in an open state according to one embodiment of the present disclosure;
FIG. 25 is a sectional view of a flow controller in a closed state according to one embodiment of the present disclosure;
FIG. 26 is a three-dimensional diagram of a flow controller according to one embodiment of the present disclosure;
FIG. 27 is a sectional view of a flow controller in a closed state according to one embodiment of the present disclosure;
FIG. 28 is a sectional view of a flow controller in an open state according to one embodiment of the present disclosure;
FIG. 29 is a schematic structural diagram of a venous sheath (flow controller in open state) according to one embodiment of the present disclosure;
FIG. 30 is a schematic structural diagram of a stent system according to one embodiment of the present disclosure;
FIG. 31 is a schematic structural diagram of a vascular stent according to one embodiment of the present disclosure;
FIG. 32 is a schematic structural diagram of a first corrugated ring of a vascular stent according to one embodiment of the present disclosure;
FIG. 33 is a schematic diagram of positions of a first corrugated ring and a second corrugated ring of a vascular stent according to one embodiment of the present disclosure;
FIG. 34 is a schematic diagram of an enclosed area defined by a first corrugated ring and a second corrugated ring of a vascular stent according to one embodiment of the present disclosure;
FIG. 35 is a schematic diagram of an overall state of a vascular stent in a compressed state according to one embodiment of the present disclosure;
FIG. 36 is a schematic diagram of a partial position of a vascular stent in a compressed state according to one embodiment of the present disclosure;
FIG. 37 is a schematic structural diagram of a vascular stent according to another embodiment of the present disclosure;
FIG. 38 is a schematic structural diagram of a vascular stent according to another embodiment of the present disclosure;
FIG. 39 is a schematic structural diagram of a vascular stent according to another embodiment of the present disclosure; and
FIG. 40 is a schematic structural diagram of a vascular stent according to another embodiment of the present disclosure.

### Detailed Description of the Disclosure

To make objectives, technical solutions, and advantages of the present disclosure clearer, the present disclosure will further be described in detail below with reference to the drawings and the embodiments. It is to be understood that specific embodiments described herein are only adopted to explain the present disclosure and not intended to limit the present disclosure.

Unless otherwise defined, all technical and scientific terms used herein have the same meanings as commonly understood by those skilled in the art to which the present disclosure belongs. Terms used in the description of the present disclosure are for the purpose of describing specific embodiments only and are not intended to limit the present disclosure. The term "and/or" as used herein includes any and all combinations of one or more of the associated listed items.

To describe a structure of a revascularization system more clearly, the terms "proximal end" and "distal end" are defined herein as commonly used terms in the field of interventional medical devices. Specifically, "distal end" refers to an end away from an operator during surgery, and "proximal end" refers to an end close to the operator during surgery.

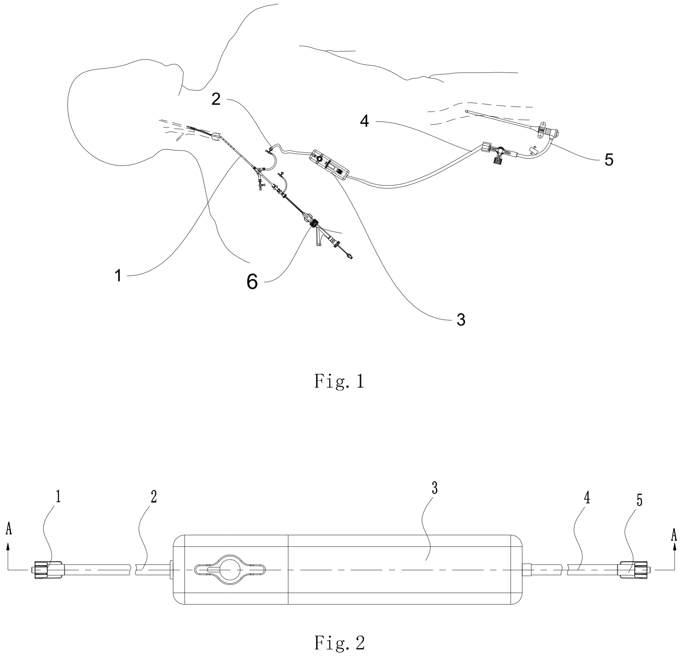

As shown in FIG. 1 and FIG. 2, a revascularization system 100 of the present disclosure includes an arterial sheath 1 (a first sheath), an inflow tube 2, a bypass assembly 3, an outflow tube 4, and a venous sheath 5 (a second sheath), which are connected in sequence. Generally, the arterial sheath 1 is configured to be connected to the carotid artery, while the venous sheath 5 is configured to be connected to the femoral vein. The inflow tube 2, the bypass assembly 3, and the outflow tube 4 collectively form a basic structure of a bypass system 200. Specifically, when bypassing is required, the blood flows from the carotid artery to a distal end of the arterial sheath 1, flows in the venous sheath 5 via the bypass assembly 3, and then flows in the vein, thereby establishing a complete revascularization system.

It should be noted that the aforementioned sites refer to connection sites commonly used in revascularization surgery, the arterial sheath 1 and the venous sheath 5 are configured to bypass the blood flow from the carotid artery into the femoral vein. However, in some specific cases, the revascularization system may also meet artery-to-artery or vein-to-vein requirements, and the blood flow is consistently directed from the first sheath to the second sheath, specifically, from the arterial sheath 1 to the venous sheath 5 in this embodiment.

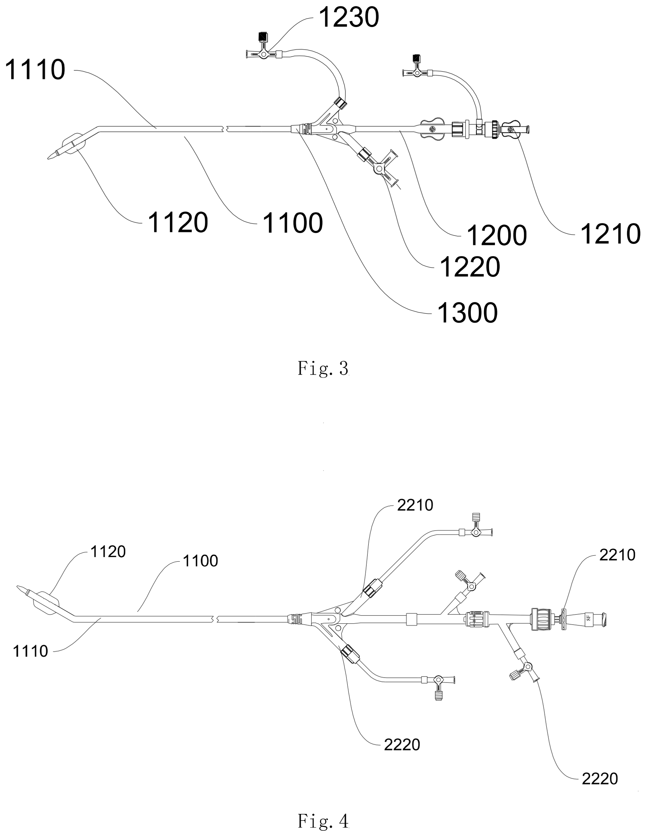

Referring to FIG. 3, the arterial sheath 1 includes a sheath 1100 and a sheath hub 1200, and further includes a dilator 1210 that is inserted into the sheath 1100 via the sheath hub 1200. The arterial sheath 1 is integrally configured to puncture and occlude the common carotid artery, introduce a stent system, and facilitate blood shunting (arterial blood extraction). A front end of the arterial sheath 1 is pre-bent to conform to a vascular pathway, thereby minimizing contact with a vessel wall and promoting central positioning of a balloon.

Overall, the sheath 1100 includes a sheath main body 1110, and the sheath hub 1200 is arranged at a proximal end of the sheath main body 1110. Specifically, the sheath hub 1200 includes a catheter seat system 1300 located at a distal end of the sheath hub 1200, and a proximal end of the sheath main body 1110 is connected with the catheter seat 1300. Generally, the catheter seat system 1300 is communicated with the sheath main body 1110 and a proximal end of the catheter seat system 1300 is communicated with the bypass assembly 3 via a bypass valve 1230. An occluding balloon 1120 is arranged on a distal side of the sheath main body 1110. When the balloon 1120 is inflated to occlude the carotid artery, blood from the carotid artery is shunted through the arterial sheath 1. Therefore, another side of the catheter seat system 1300 is communicated with a balloon inflation valve 1220. In this embodiment, the balloon inflation valve 1220 is provided with an extension tube to prevent the hand of an operator from being exposed to radiation when inflating the balloon 1120 at the front end of the sheath.

In another embodiment, referring to FIG. 4, a sheath 2100 includes a sheath main body 2110, and a sheath hub 2200 is arranged at a proximal end of the sheath main body 2110. Specifically, the sheath hub 2200 includes a plurality of branches, a branch port of each branch serves as a connection point for different components. In this embodiment, the sheath hub 2200 is provided with one hemostatic valve 2210, one bypass valve 2222, and a corresponding balloon inflation valve 2230. In addition, to facilitate delivery more effectively, this embodiment includes not only one angiography port 2221, but also one additional front-end angiography port 2220. In this way, in an initial state, angiographic imaging is performed on a front end of the sheath by using the front-end angiography port 2220 to determine a position of the sheath and vessel dimensions. After occlusion is completed, angiographic imaging is performed via the angiography port 2221 to determine whether occlusion is completed, thereby maximizing operational accuracy.

The front end of the sheath 1100 is pre-bent (or adopts an adjustable bending manner) to facilitate insertion of the sheath 1100 into the common carotid artery. At least two radiopaque rings (not shown in the figure) are arranged in the balloon at a distal end of the sheath 1100 to indicate a balloon position. Depth markings (not shown in the figure) are also provided on a surface of the sheath 1100 to monitor a puncture depth during puncturing.

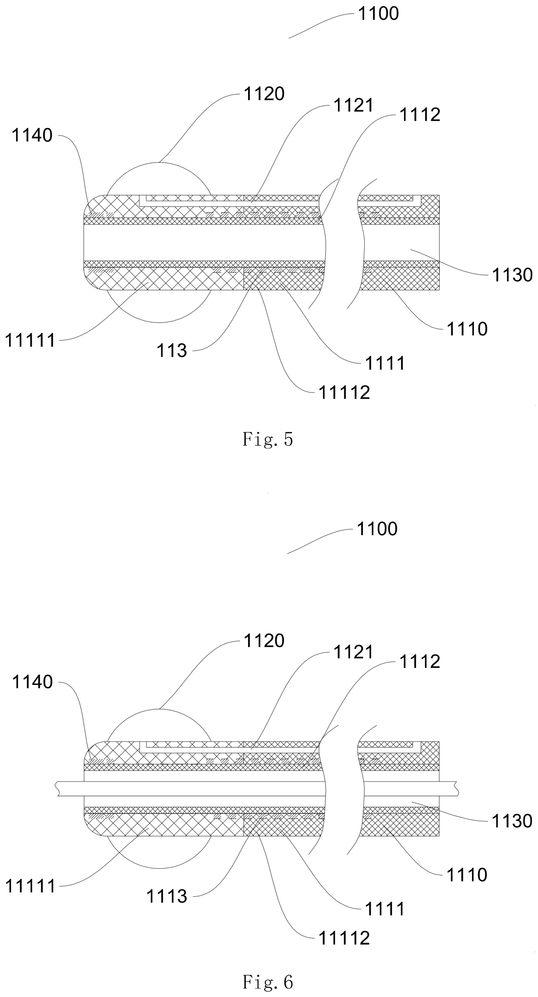

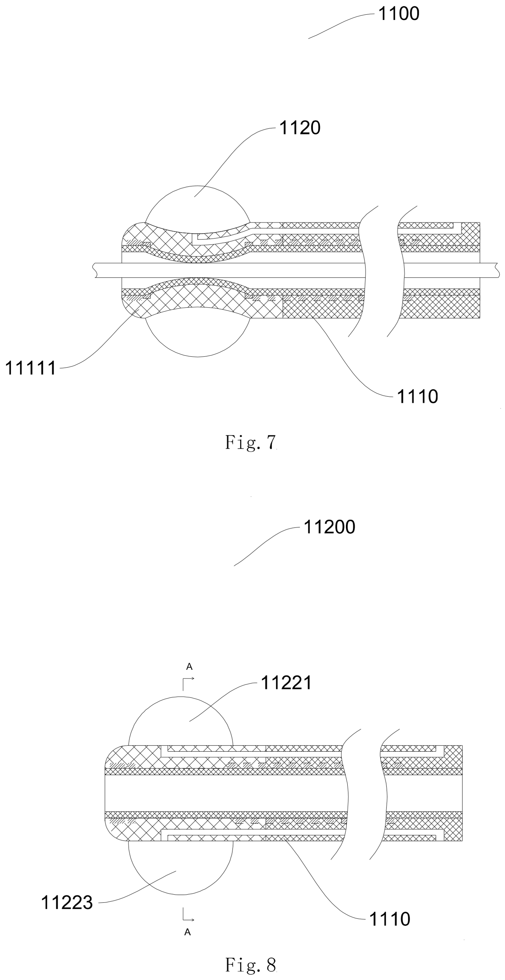

Regarding the sheath 1100, a plurality of embodiments are provided, as detailed below:
Referring to FIG. 5 to FIG. 7, FIG. 6 is a schematic diagram of a first operational state of the sheath 1100, and FIG. 7 is a schematic diagram of a second operational state of the sheath 1100. In the first operational state, the sheath 1100 is provided with a guide wire or a dilator. When the sheath switches from the first operational state to the second operational state, the balloon 1120 in the sheath 1100 is inflated.

In this embodiment, the sheath 1100 includes a tubular main body 1110. The main body 1110 includes an outer sheath layer 1111, an inner sheath layer 1112, the balloon 1120 located mostly outside the outer sheath layer 1111, and a lumen 1130 formed on an inner side of the inner sheath layer 1112 for delivering a medical device. The main body 1110 primarily serves as a channel for device delivery. Since the main body 1110 needs to be introduced into a human body, the outer sheath layer 1111 of the main body 1110 is required to exhibit good biocompatibility and certain strength. Accordingly, in this embodiment, the outer sheath layer 1111 is preferably made of polyether block amide (PEBAX) material. A middle channel formed by the inner sheath layer 1112 serves as a device delivery channel. To ensure smooth device delivery, the inner sheath layer 1112 preferably includes polytetrafluoroethylene (PTFE) film. To enhance the strength of the inner sheath layer 1112, prevent the formation of folds that may impede device delivery, and avoid excessive strength increase from compromising the flexibility of the main body 1110, a spring tube 1113 is wound around an outer surface of the inner sheath layer 1112. The outer sheath layer 1111 is then slidably covered over both the inner sheath layer 1112 and the spring tube 1113.

In this embodiment, the balloon 1120 is arranged at a distal end of the main body 1110. The balloon 1120 is a semi-compliant balloon. As the inflation pressure increases, the balloon 1120 expands in diameter, is closely adhered to a vessel inner wall, and effectively occludes the vessel to block blood flow. When the balloon 1120 is inflated, the main body 1110 is located in the center of the balloon 1120, such that the main body 1110 does not contact the vessel inner wall during occlusion of the vessel, thereby protecting the vessel from injury.

In this embodiment, as the balloon 1120 is gradually inflated to block blood flow, a device can still pass through smoothly. When an internal inflation pressure of the balloon 1120 exceeds a specified value, the internal pressure of the balloon 1120 causes the main body 1110 to deform and press against the device, thereby achieving fixation of the device and hemostasis. It should be noted that in this embodiment, an inflation lumen 1121 of the balloon 1120 extends through the interior of the outer sheath layer 1111. Compared with conventional designs in which the inflation lumen of the balloon is additionally covered on an exterior of the sheath, this design results in a significantly smaller overall size. Specifically, while maintaining a favorable inflation rate, the main body 1110 can achieve a smaller outer diameter and a larger inner diameter, thereby maximizing an available space for device delivery.

In this embodiment, a radiopaque ring (not shown in the figure) is arranged at the distal end of the main body 1110 and facilitates positioning of the sheath 1100 after implantation.

Additionally, in this embodiment, the proximal side and the distal side of the main body 1110 are configured to have different strengths. Specifically, the distal side of the main body 1110 is more flexible to avoid vessel injury during device delivery and insertion into the human body, while the proximal side of the main body 1110 provides a higher support strength to maintain the overall strength of the main body 1110 and enable effective transmission of force from the proximal end to the distal end when moving toward a predetermined position. Preferably, the outer sheath layer 1111 of the main body 1110 includes a first section 11111 and a second section 11112, wherein the first section 11111 is made of PEBAX 35D material, and the second section 11112 is made of PEBAX 63D material.

In this embodiment, the balloon 1120 is expanded by injecting a medium (such as a contrast agent) into the inflation lumen 1121 of the balloon 1120 to occlude the vessel, and simultaneously device delivery and blood flow shunting are achieved through the lumen 1130.

Referring to FIG. 5, when the inflation pressure of the balloon 1120 gradually increases but remains below a preset value, the balloon 1120 expands to block blood flow. In this case, the balloon 1120 gradually expands to fill a gap between the main body 1110 and the vessel, while allowing the device in the main body 1110 to pass through normally. It should be noted that the preset value is determined based on the pressure strength of the balloon and the overall strength of the distal end (i.e., the first section 11111) of the main body 1110.

When the preset value is reached, it means that the internal pressure of the balloon 1120 can cause a distal section of the main body 1110 to deform, as shown in FIG. 6. At this moment, the balloon 1120 not only fills the gap between the main body 1110 and the vessel, but also forces a corresponding position of the distal side of the main body 1110 to deform inward, thereby causing the inner wall of the main body 1110 to press against the device, and achieving both fixation of the device and blocking of blood flow.

Since the expansion volume of the balloon 1120 can be controlled, when this embodiment is applied to blood flow shunting, i.e., when the lumen 1130 serves as a channel for blood flow shunting, it can achieve the following: when the balloon 1120 just expands to fill the gap between the main body 1110 and the vessel, the lumen 1130 is communicated with the vessel to participate in blood flow shunting (i.e., cooperating for reverse blood flow); and as the balloon 1120 continues to inflate, the internal pressure increases and causes the main body 1110 to deform, the diameter of the lumen 1130 is gradually reduced, thereby reducing the blood flow velocity, and this process continuous until the flow stops completely. In this way, the blood flow shunting velocity is controlled, and the need for using intermediary components to regulate a flow velocity is eliminated.

In this embodiment, to prevent excessive pressure from the inflated balloon 1120 from damaging the vessel wall, preferably, no spring tube 1113 is placed in an area corresponding to the balloon 1120 of the inner sheath layer 1112. In other words, a strength of the main body 1110 in an area of the balloon 1120 is less than that of the main body 1110 in other areas.

In another embodiment, an auxiliary channel is arranged between the balloon 1120 and the inner sheath layer 1112, that is, the balloon 1120 is communicated with an outer surface of the inner sheath layer 1112. When the balloon 1120 is inflated, the pressure of the balloon 1120 can directly act on the inner sheath layer 1112, thereby preventing deformation of the outer sheath layer 1111 from causing deformation of other areas, and reducing the difficulty of causing the main body 1110 to deform.

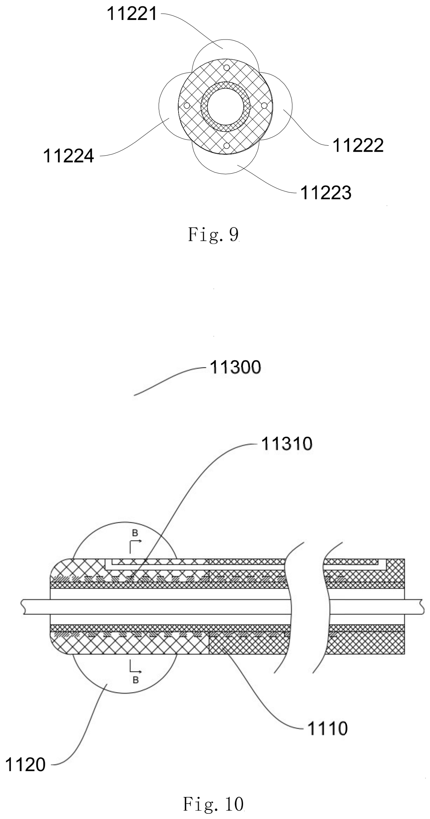

In another alternative embodiment, a plurality of balloons are arranged at the distal end of the main body 1110 in a circumferential direction. Referring to FIG. 8 and FIG. 9, FIG. 9 is a schematic cross-sectional view of the position A-A in FIG. 8 from left to right. A plurality of balloons are arranged at the distal end of the main body 1110 of the sheath 1100 in a circumferential direction, including a balloon 11221, a balloon 11222, a balloon 11223, and a balloon 11224. Each balloon has a separate inflation lumen, so the balloon 11221, the balloon 11222, the balloon 11223, and the balloon 11224 can be controlled individually. By inflating different balloons, the fixation of the device in a balloon sheath 11200 and a reverse blood flow rate can be flexibly controlled, and this configuration offers a good adaptability compared with a single-balloon configuration.

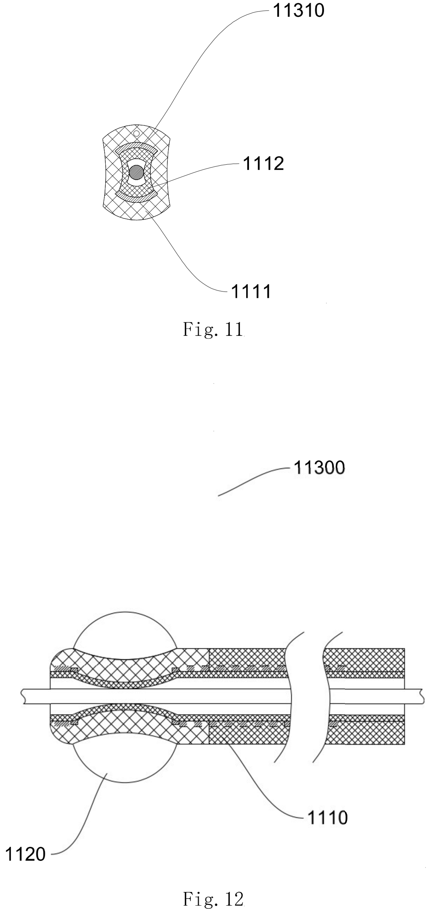

In another alternative embodiment, an intermediate layer is arranged at the distal end of the main body 1110. Referring to FIG. 10 to FIG.12, FIG. 10 is a schematic diagram of a first sectional structure of a balloon sheath 11300, FIG. 11 is a schematic cross-sectional view of the position B-B in FIG. 10 from left to right, and FIG. 12 is a schematic diagram of a second sectional structure of a balloon sheath 11300. Both the first section and the second section are longitudinal sections, and the first section is perpendicular to the second section. FIG. 10 and FIG. 11 show the balloon 1120 in an inflated state, with the purpose of illustrating a coverage area and function of the intermediate layer 11310.

The intermediate layer 11310 partially covers a surface of the inner sheath layer 1112 in a circumferential direction, thereby reinforcing the strength of the inner sheath layer 1112 in some areas. In this embodiment, a spring tube may be selected, and its specific distribution position is shown in FIG. 10.

It should be noted that, due to the presence of the intermediate layer 11310, when the balloon 1120 is subjected to a relatively large inflation pressure, an area of the main body 1110 that deforms inward is an area not covered by the intermediate layer 11310. This is because the intermediate layer 11310 has supporting strength and can counteract the pressure exerted by the balloon 1120. The purpose of this design is to reserve a space between an inner wall of the balloon sheath 11300 (an area supported by the intermediate layer) and a device, allowing reverse flow of blood, while enabling other areas of the main body 1110 to deform normally. This ensures that both fixation of the device and blood flow shunting are achieved.

Generally, as the inflation pressure gradually increases, the balloon expands to block blood flow, and the device can pass through normally. When the inflation pressure exceeds a specified value, the balloon compresses part of the sheath longitudinally, causing a longitudinal inner wall of the sheath (without spring support in longitudinal direction) to press against the device, while a lateral inner wall of the sheath (with spring support in lateral direction) creates a gap with the device, allowing reverse flow of blood. This ensures that both fixation of the device and blood flow shunting are achieved.

In this embodiment, the intermediate layer 11310 may be selected from flexible elastic components such as a spring strip or a mesh sheet. The purpose is not necessarily to always maintain a gap between the inner wall of the balloon sheath 11300 (an area supported by the intermediate layer 11310) and the device, but rather to ensure that an area with the intermediate layer 11310 requires relatively higher pressure to deform compared to an area without the intermediate layer 11310. This creates staged deformation, for example: when a medium is injected into the balloon 1120, the pressure gradually increases to a first stage, where the balloon initially blocks the gap between an outer side of the main body 1110 and the vessel; when the pressure of the balloon 1120 is increased to a second stage, the area without the intermediate layer 11310 of the main body 1110 deforms, and a gap between the inner wall of the balloon sheath 11300 (the area supported by the intermediate layer) and the device is formed to allow reverse flow of blood, and other areas can still deform normally, thereby achieving both fixation of the device and blood flow shunting; and when the pressure of the balloon 1120 is increased to a third stage, the area with the intermediate layer 11310 also begins to deform, and the gap between the inner wall of the main body 1110 and the device is filled, thereby fixing the device and stopping blood flow shunting.

It should be noted that if the balloon 1120 directly acts on the inner sheath layer 1112 (with no deformation of the outer sheath layer 1111), the intermediate layer 11310 is required to be correspondingly arranged on the inner sheath layer 1112.

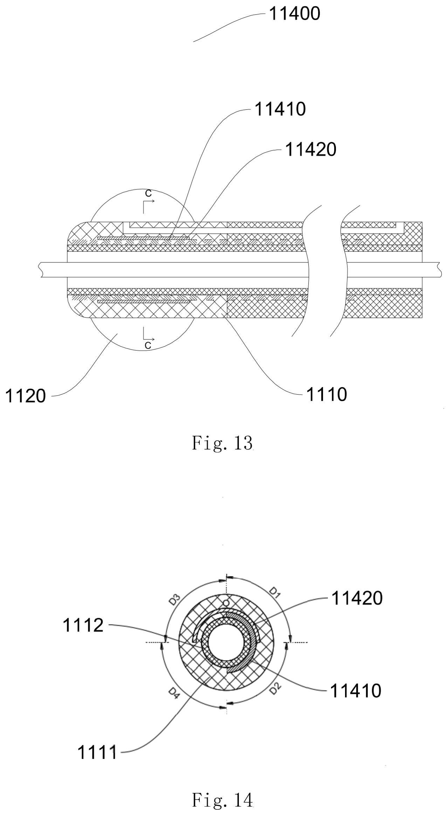

In another alternative embodiment, a plurality of intermediate layers are arranged. Referring to FIG. 13 and FIG. 14, FIG. 14 is a schematic sectional view of the position C-C in FIG. 13 from left to right. In this embodiment, at least a first intermediate layer 11410 and a second intermediate layer 11420 are arranged in the main body 1110, and partially overlap and cover the main body 1110 in a circumferential direction.

In this embodiment, the first intermediate layer 11410 is a spring tube structure, and the second intermediate layer 11420 is a spring strip structure. In this embodiment, the strength of the first intermediate layer 11410 is greater than that of the second intermediate layer 11420. When the two layers are distributed as shown in FIG. 13 and FIG. 14, at least the following four areas are formed:
a first area D1, including both the first intermediate layer 11410 and the second intermediate layer 11420, and requiring the greatest deformation pressure;
a second area D2: only including the first intermediate layer 11410, and requiring deformation pressure less than that of the first area D 1;
a third area D3: only including the second intermediate layer 11420, and requiring deformation pressure less than that of the second area D2; and
a fourth area D4: not covered by any intermediate layer and requiring the least deformation pressure.

With this design, an inflation phase of the balloon 1120 can be divided into the following four stages.

In the first stage, the medium is injected into the balloon 1120 to gradually increase the pressure, the balloon completes the occlusion of the gap between the outer side of the main body 1110 and the vessel, and at this moment, blood is allowed to be shunted from the inside of the main body 1110 in reverse flow.

In the second stage, the inflation pressure is further applied to the balloon 1120, the fourth area D4 of the main body 1110 deforms, the main body 100 in the fourth area D4 moves inward, the inner wall of the main body 100 in other areas (supported by the intermediate layers) is still spaced from the device, thereby reducing the space for reverse blood flow shunting; and as a result, the flow velocity reverse blood flow shunting decreases, the velocity of blood flow shunting is controlled to a value defined in the second stage, and if portions of the fourth area D4 are distributed relative to each other, the fourth area D4 also has the function of fixing the device (by pressing the device at a relative position).

In the third stage, the inflation pressure is applied further to the balloon 1120 on the basis of the second stage, the third area D3 deforms, the space for reverse blood flow shunting is even reduced, the flow velocity of reverse blood flow shunting is further decreased, and the flow velocity of blood flow shunting is further controlled to a value defined in the third phase.

In the fourth stage, the inflation pressure is applied further to the balloon 1120 on the basis of the third stage, the second area D2 deforms, the space for reverse blood flow shunting is even reduced, the flow velocity of reverse blood flow shunting is further decreased, and the flow velocity of blood flow shunting is further controlled to a value defined in the fourth phase.

In the fifth stage, the inflation pressure is applied further to the balloon 1120 on the basis of the fourth stage, the first area D1 deforms, the space for reverse blood flow is even reduced, the flow velocity of reverse blood flow shunting is further decreased until the main body 1110 in the first area D1 is pressed against the device; at this point, the inner wall of the main body 1110 in the first area D1 is tightly pressed against the device, thereby achieving complete fixation of the device; and at this moment, the flow velocity of blood flow shunting is nearly zero, and the blood flow shunting stops.

Therefore, as described in this embodiment, a more complex multi-stage regulation of reverse blood flow shunting is achieved by partially overlapping the two intermediate layers.

In another embodiment, the first intermediate layer 11410 and the second intermediate layer 11420 have identical structures, but have different thicknesses. This configuration can also achieve similar technical effects as in this embodiment. However, an overall thickness of a tube body may be increased or a center of gravity of the distal end of the main body 1110 may deviate from the axis.

In another embodiment, areas covered by the first intermediate layer 11410 and the second intermediate layer 11420 do not overlap at all, that is, the first intermediate layer 11410 and the second intermediate layer 11420 are completely separated from each other.

In another embodiment, the main body 1110 includes a plurality of first intermediate layers 11410 and/or a plurality of second intermediate layers 11420.

In another embodiment, the main body 1110 includes a plurality of first intermediate layers 11410 and/or a plurality of second intermediate layers 11420, where the first intermediate layers 11410 extend in an axial direction, and the second intermediate layers 11420 extend in a circumferential direction. In this way, a plurality of areas which have different strengths, are uniformly distributed, and deformation with the balloon 1120 may be formed.

The catheter seat 1300, in addition to having a communication function, also has a special design, as detailed below.

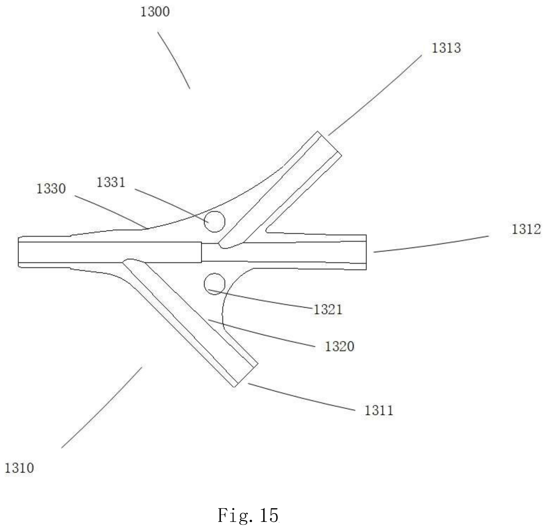

Referring to FIG. 15, the catheter seat 1300 includes a catheter seat body 1310. The catheter seat body 1310 includes a first cavity 1311, a second cavity 1312, and a third cavity 1313. Tail ends of the first cavity 1311, the second cavity 1312, and the third cavity 1313 are all provided with Luer connectors. The second cavity 1312 extends linearly in a catheter direction, while the first cavity 1311 and the third cavity 1313 are respectively located on two sides of the second cavity 1312. When the catheter seat 1300 is applied in carotid revascularization, the first cavity 1311 is communicated with the balloon 1120 to inflate and deflate the balloon, i.e., the first cavity 1311 is communicated with the inflation lumen of the balloon 1120. The second cavity 1312 serves as an accommodating cavity to accommodate entry and exit of the guide wire or the dilator, thereby achieving interventional device delivery. The third cavity 1313 serves as a blood shunting channel to facilitate blood transfer.

In another embodiment, the second cavity 1312 serves as an accommodating cavity to accommodate entry and exit of the guide wire or the dilator, thereby achieving interventional device delivery, and a side branch opening of the second cavity 1312 is communicated with one retrograde flow channel for blood circulation. The third cavity 1313 serves as an angiography lumen.

In another embodiment, the second cavity 1312 serves as an accommodating cavity to accommodate entry and exit of the guide wire or dilator, thereby achieving interventional device delivery, and a side branch opening of the second cavity 1312 is communicated with one retrograde flow channel for blood circulation. Additionally, a side branch of the second cavity 1312 is also communicated with a fourth cavity (not shown in the figure), and both the fourth cavity and the third cavity serve as angiography lumens. The third cavity 1313 is primarily responsible for angiography for a rear end area (a proximal side) of the balloon, while the fourth cavity is primarily responsible for angiography for a front end area (a distal side, may also be referred to as a distal or front end of the sheath) of the balloon. Specifically, an operator can confirm an implantation position and vessel dimensions through angiography at the front end of the balloon (sheath). After the balloon is inflated for occlusion, the operator determines whether the occlusion is complete through angiography for the rear end area of the balloon.

In this embodiment, further, a first connection plate 1320 is arranged between the first cavity 1311 and the second cavity 1312. The first connection plate 1320 is provided with at least one suture hole 1321. Similarly, a second connection plate 1330 is arranged between the third cavity 1313 and the second cavity 1312. The second connection plate 1330 is provided with at least one suture hole 1331. The suture hole 1321 and the suture hole 1331 fix the catheter seat body 1310 to skin. This design in this embodiment shortens the overall length of the catheter seat system, also reduces the overall length of the catheter system, and minimizes dimensions of an interventional implantation system. Additionally, the fixation position is close to the catheter seat body 1310, which can help avoid excessive minor movement of the catheter seat body 1310.

In this embodiment, an edge of the first connection plate 1320 is curved. To make the suturing position of the suture hole 1321 as close as possible to the catheter seat body for a good fixation effect, an edge of the first connection plate 1320 is recessed toward the connection position between the first cavity 1311 and the second cavity 1312 and a line connecting the point of maximum curvature of the edge of the first connection plate 1320 and the position closest to the edge of the first connection plate 1320 of the suture hole 1321 passes through the center of the suture hole 1321. In this state, the path traveled by a suture when the suture fixes the catheter seat body 1310 via the suture hole 1321 and the edge of the first connection plate 1320 is the shortest.

Similarly, in this embodiment, an edge of the second connection plate 1330 is curved. To make the suturing position of the suture hole 1331 as close as possible to the catheter seat body for a good fixation effect, an edge of the second connection plate 1330 is recessed toward the connection position between the third cavity 1313 and the second cavity 1312 and a line connecting a point of maximum curvature of the edge of the second connection plate 1330 and the position closest to the edge of the second connection plate 1330 of the suture hole 1331 passes through the center of the suture hole 1331. In this state, the path traveled by a suture when the suture fixes the catheter seat body 1310 via the suture hole 1331 and the edge of the second connection plate 1330 is the shortest.

Preferably, a line connecting the center of the suture hole 1321 and the center of the suture hole 1331 is essentially perpendicular to the axis of the second cavity 1312.

Preferably, both the first connection plate 1320 and the second connection plate 1330 are arranged on a side close to a human body of the catheter seat body 1310. Further, the first connection plate 1320 and the second connection plate 1330 are coplanar with at least one side of the first cavity 1311, second cavity 1312, and third cavity 1313. In other words, the first connection plate 1320 and the second connection plate 1330 are not arranged in a plane formed by axes of the first cavity 1311, second cavity 1312, and third cavity 1313, but rather in a plane that is tangent to the first cavity 1311, the second cavity 1312, and the third cavity 1313. Side surfaces of the first connection plate 1320 and the second connection plate 1330 are coplanar with the housing of the first cavity 1311, second cavity 1312, and third cavity 1313, thereby reducing the impact of a diameter of the cavities of the catheter seat body 1310 and ensuring that the first connection plate 1320 and the second connection plates 1330 adhere tightly to the skin.

In this embodiment, an included angle between the first cavity 1311 and the second cavity 1312 is between 30° and 60°, preferably 45°, and an included angle between the third cavity 1313 and the second cavity 1312 is between 30° and 90°, preferably 45°.

It should be noted that in this embodiment, the connection position between the first cavity 1311 and the second cavity 1312 is different from the connection position between the third cavity 1313 and the second cavity 1312, and the edge of the first connection plate 1320 is located at a proximal end of the connection position between the first cavity 1311 and the second cavity 1312, while the edge of the second connection plate 1330 is located at a distal end of the connection position between the third cavity 1313 and the second cavity 1312.

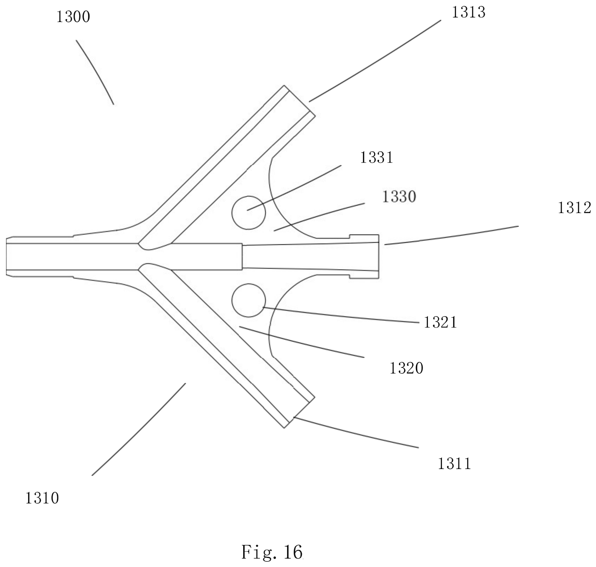

In another embodiment, referring to FIG. 16, which is a schematic structural diagram of a variant of the catheter seat 1300. In FIG. 16, the connection position between the first cavity 1311 and the second cavity 1312, and the connection position between the third cavity 1313 and the second cavity 1312 are at the same position in an axial direction. In this case, to make the line connecting the center of the suture hole 1321 and the center of the suture hole 1331 as perpendicular as possible to the axis of the second cavity 1312, preferably, the edge of the first connection plate 1320 is located at a proximal end of the connection position between the first cavity 1311 and the second cavity 1312, and the edge of the second connection plate 1330 is located at a proximal end of the connection position between the third cavity 1313 and the second cavity 1312.

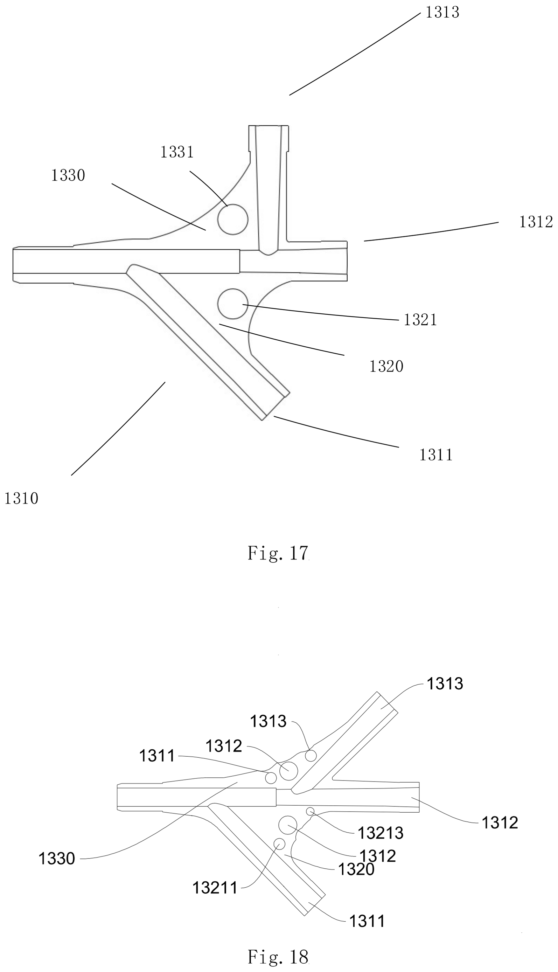

In another embodiment, referring to FIG. 17, which is a schematic structural diagram of another variant of the catheter seat 1300. In FIG. 17, the connection position between the first cavity 1311 and the second cavity 1312 is different from the connection position between the third cavity 1313 and the second cavity 1312, but the included angle between the third cavity 1313 and the second cavity 1312 is 90°. In this case, to make the line connecting the center of the suture hole 1321 and the center of the suture hole 1331 as perpendicular as possible to the axis of the second cavity 1312, preferably, the edge of the first connection plate 1320 is located at a proximal end of the connection position between the first cavity 1311 and the second cavity 1312, and the edge of the second connection plate 1330 is located at a distal end of the connection position between the third cavity 1313 and the second cavity 1312.

In another alternative embodiment, a plurality of suture holes are formed in the connection plate. Referring to FIG. 18, the first connection plate 1320 is arranged between the first cavity 1311 and the second cavity 1312, and a first suture hole 13211, a second suture hole 13212, and a third suture hole 13213 are respectively formed in the first connection plate 1320; and the second connection plate 1330 is arranged between the third cavity 1313 and the second cavity 1312, and a fourth suture hole 13311, a fifth suture hole 13312, and a sixth suture hole 13313 are respectively formed in the second connection plate 1330. This configuration is intended to satisfy certain conditions where a plurality of suturing positions are required to enhance fixation, or where combinations of the plurality of suture holes are needed to meet different suturing requirements.

In this embodiment, the edge of the first connection plate 1320 is curved. To make the suturing positions of the first suture hole 13211, the second suture hole 13212, and the third suture hole 13213 as close as possible to the catheter seat body for a good fixation effect, the edge of the first connection plate 1320 is recessed toward the connection position between the first cavity 1311 and the second cavity 1312; in an area close to the suture hole 13211, a line connecting a point of maximum curvature of the edge of the first connection plate 1320 and the position closest to the edge of the first connection plate 1320 of the suture hole 13211 passes through the center of the suture hole 13211; in an area close to the suture hole 13212, a line connecting a point of maximum curvature of the edge of the first connection plate 1320 and the position closest to the edge of the first connection plate 1320 of the suture hole 13212 passes through the center of the suture hole 13212; and in an area close to the suture hole 13213, a line connecting a point of maximum curvature of the edge of the first connection plate 1320 and the position closest to the edge of the first connection plate 1320 of the suture hole 13213 passes through the center of the suture hole 13213. Overall, the edge of the first connection plate 1320 presents an undulating curved profile.

In this embodiment, the edge of the second connection plate 1330 is curved. To make the suturing positions of the fourth suture hole 13311, the fifth suture hole 13312, and the sixth suture hole 13313 as close as possible to the catheter seat body for a good fixation effect, the edge of the second connection plate 1330 is recessed toward the connection position between the third cavity 1313 and the second cavity 1312; in an area close to the suture hole 13311, a line connecting a point of maximum curvature of the edge of the second connection plate 1330 and the position closest to the edge of the second connection plate 1330 of the suture hole 13311 passes through the center of the suture hole 13311; in an area close to the suture hole 13312, a line connecting a point of maximum curvature of the edge of the second connection plate 1330 and the position closest to the edge of the second connection plate 1330 of the suture hole 13312 passes through the center of the suture hole 13312; and in an area close to the suture hole 13313, a line connecting a point of maximum curvature of the edge of the second connection plate 1330 and the position closest to the edge of the second connection plate 1330 of the suture hole 13313 passes through the center of the suture hole 13313. Overall, the edge of the second connection plate 1330 presents an undulating curved profile.

Preferably, a line connecting the center of the suture hole 13212 and the center of the suture hole 335 is substantially perpendicular to the axis of the second cavity 1312. Additionally, a line connecting the center of the suture hole 1321 and the center of the suture hole 334 is substantially perpendicular to the axis of the second cavity 1312, or a line connecting the center of the suture hole 13213 and the center of the suture hole 336 is also substantially perpendicular to the axis of the second cavity 1312.

In another alternative embodiment, the connection plate is detachable. Specifically, the second connection plate 1330 is secured between the third cavity 1313 and the second cavity 1312 through a slot structure, whereby connection plates of different shapes may be selected according to implantation sites to better adhere to the skin. In another embodiment, a stress tube is arranged at a proximal end of the catheter seat 1300 and has a relatively high strength (but lower than the hardness of the sheath) so as to prevent folding at the connection position when the sheath is bent.

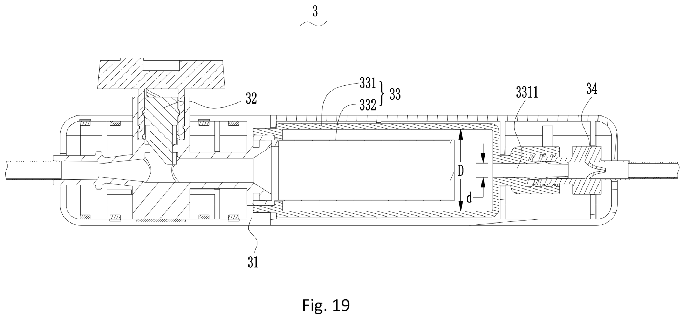

Regarding the bypass assembly 3, during stent implantation, the bypass assembly 3 is capable of controlling the blood flow velocity. Moreover, a one-way valve arranged in the bypass assembly 3 allows the blood to flow extracorporeally toward a vein in a specific direction. Specifically:
Referring to FIG. 19, the bypass assembly 3 includes a housing 31 and a flow controller 32, a blood filter 33, and a one-way check valve 34, which are sequentially connected in the housing 31. The flow controller 32 is connected to an end of the inflow tube 2 away from the arterial sheath 1, and the one-way check valve 34 is connected to an end of the outflow tube 4 away from the venous sheath 5.

Further, the one-way check valve 34 is connected between the blood filter 33 and the outflow tube 4.

The flow controller 32 includes a main body element 321 with a flow channel 3211 and a flow control element 322. The flow control element 322 is at least partially accommodated in the main body element 321 and is movably connected to the flow channel 3211. The flow control element 322 can reduce or increase a flow area of the flow channel 3211 under an external force.

Before a stent is placed at the site of carotid artery stenosis, the arterial sheath 1 of the present disclosure is placed in the common carotid artery, and the venous sheath 5 is placed in the femoral vein. A pressure difference is formed between a high-pressure arterial blood flow in the brain and a low-pressure venous blood flow in the femoral vein, causing blood from the carotid artery to flow in reverse to the femoral vein via the revascularization system. After the revascularization system is established, operations such as pre-dilation of the carotid artery stenosis and placement of a self-expanding stent can be performed. Thrombus that detaches during surgery is intercepted by the blood filter when it flows through with the reverse blood flow, thereby preventing it from entering the body with the blood. The flow controller 32 enables the operator to adjust a flow rate of the reverse blood flow in real time.

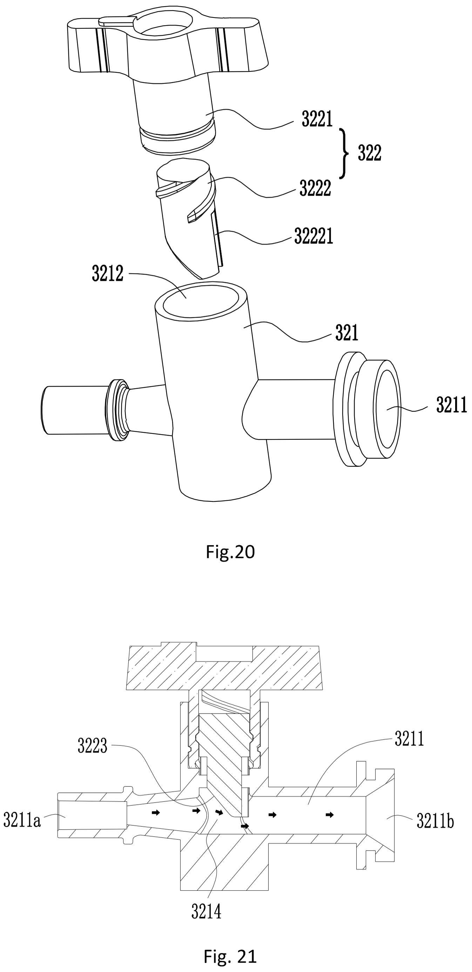

In this embodiment, as shown in FIG. 20 and FIG. 21, the main body element 321 further includes a movable channel 3212 communicated with the flow channel 3211. The flow control element 322 is movably arranged in the movable channel 3212. Under an external force, the distal end of the flow control element 322 moves toward the flow channel 3211. The distal end of the flow control element 322 extends out of the movable channel 3212 and enters the flow channel 3211, thereby obstructing the flow channel 3211 to a certain extent, reducing the flow area of the flow channel 3211, and further reducing the flow rate of the revascularization system. Conversely, under an external force, the distal end of the flow control element 322 moves away from the flow channel 3211, thereby gradually reducing the obstruction to the flow channel 3211 and increasing the flow rate of the revascularization system. This enables stepless adjustment of the blood flow velocity to avoid causing discomfort to a patient.

In other embodiments, the main body element may not include the movable channel, and instead, through holes may be directly formed in a side wall of the flow channel, the flow control element extends through the through holes and moves along the through holes to adjust the flow area of the flow channel.

For ease of description, FIG. 20 shows an inlet end 3211a and an outlet end 3211b of the flow channel 3211. The inlet end 3211a is close to the inflow tube 2, while the outlet end 3211b is far away from the inflow tube 2. In actual use, the blood flow enters the flow channel 3211 via the inlet end 3211a and exits via the outlet end 3211b, and arrows in FIG. 21 indicate the direction of blood flow.

The flow control element 322 includes a guiding surface 3223. The guiding surface is either an inclined or curved surface directed towards the inlet end 3211a, and defines, together with an inner surface of the flow channel 3211, one flow-regulating channel 3214 that gradually tapers from the inlet end 3211a to the outlet end 3211b.

In this embodiment, the distal end of the flow control element 322 is provided with an inclined surface, which forms the guiding surface 3223. Since the revascularization system of the present disclosure is used in conjunction with carotid artery stent implantation surgery, thrombus present in the carotid artery inevitably detaches during the surgery and flows through the revascularization system. Due to the presence of thrombus, any obstruction or curvature in the flow channel 3211 increases the risk of thrombus accumulation, which in turn poses a risk of blocking the flow controller 32.

Therefore, the flow control element 322 of the present disclosure is provided with the guiding surface 3223, which makes it difficult for thrombus to accumulate on the guiding surface inclined relative to a blood flow direction. Even when the flow controller 32 is in a low-flow state, thrombus is less likely to accumulate into large masses at the flow controller 32, thereby ensuring the smoothness of the revascularization system.

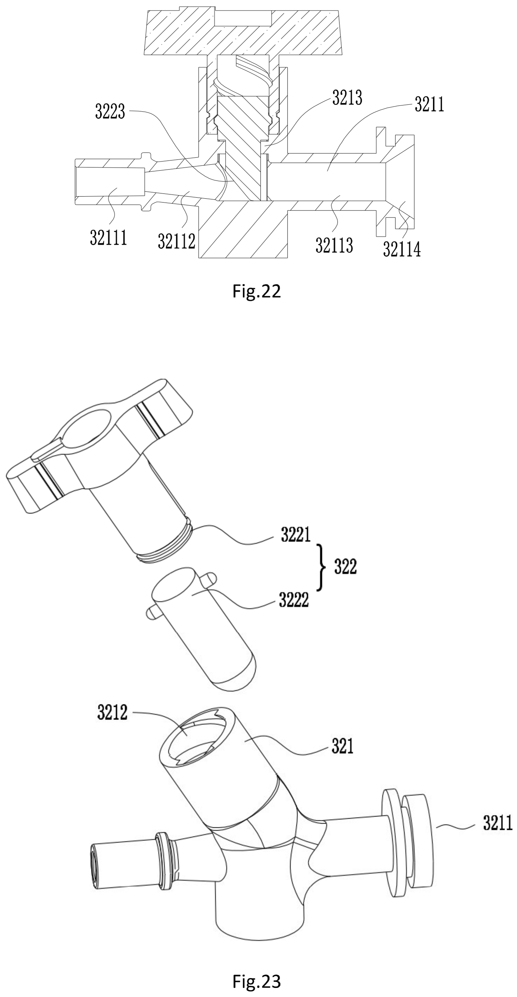

As shown in FIG. 22, the flow channel 3211 includes a connection section 32111, a constricted section 32112, and a main section 32113, which are sequentially connected from the inlet end 3211a to the outlet end 3211b. The movable channel 3212 is arranged on the main section 32111 and is close to the constricted section 32112. The inner diameter of the constricted section 32112 is smaller than that of the connection section 32111 and the main section 32113. The connection section 32111 is used to be connected with the inflow tube 2, and a tail end of the connection section 32111 is connected with the constricted section 32112 with a smaller inner diameter. In this way, blood passes through the constricted section 32112 at an increased flow velocity, and the increased flow velocity allows the blood to flow more quickly through the flow-regulating channel 3214, thereby further preventing thrombus from accumulating on an inflow side of the flow control element 322.

As shown in FIG. 21, a distal end face of the flow control element 322 is matched with the shape of the bottom of the flow channel 3211. Thus, the distal end of the flow control element 322 can be closely fitted with the flow channel 3211, that is, the flow controller 32 is in a closed state, and the flow controller 32 has a hemostatic function.

Specifically, in this embodiment, referring to FIG. 20 to FIG. 22, a side face of a sliding member 3222 is provided with a sliding groove 32221, and a bump 3213 is arranged inside the movable channel 3212. The bump 3213 cooperates with the sliding groove 32221 in a sliding way to enable the sliding member 3222 to move axially within the movable channel 3212. An outer wall of a knob 3221 is provided with an annular groove, and an inner wall of the movable channel 3212 has a protrusion that is engaged with the annular groove, so the knob 3221 can rotate along the movable channel 3212 but is restricted from axial movement. An accommodating cavity is arranged at a distal end of the knob 3221, and the sliding member 3222 is inserted into the accommodating cavity and is in threaded connection with the knob 3221. Since the sliding member 3222 cooperates with the movable channel 3212 in a sliding manner, when the knob 3221 is rotated under an external force, the sliding member 3222, which is in threaded connection with the knob 3221, can only move in an axial direction of the movable channel 3212, thereby ensuring that the guiding surface is always oriented toward the inlet end 3211a of the flow channel 3211. Furthermore, because the sliding member 3222, which is in direct contact with the blood, is accommodated in the accommodating cavity of the knob 3221, the operator can rotate the knob 3221 to move the sliding member 3222 in the movable channel 3212, and contamination of the blood in the movable channel 3212 by the external environment can be avoided.

In other embodiments, the outer wall of the knob 3221 has a protrusion, and an inner wall of the movable channel 3212 may have an annular groove that is engaged with the protrusion. Alternatively, the outer wall of the movable channel 3212 has an annular groove, and the knob 3221 has a protrusion that is engaged with the annular groove. As long as the knob 3221 can rotate along the movable channel 3212 but cannot move in the axial direction, the same function is achieved. In other embodiments, the proximal end of the sliding member 3222 has a cavity, and the distal end of the knob 3221 is inserted into the cavity and in threaded connection with the sliding member 3222. It should be noted that the "proximal end" refers to the end of the flow control element close to the operator in the axial direction, and the "distal end" refers to the end of the flow control element farther away from the operator in the axial direction.

Referring to FIG. 19 again, the blood filter 33 of this embodiment includes a filter cartridge 331 and a filter mesh 332 arranged in the filter cartridge 331. The filter cartridge 331 has a tubular structure with an opening at both ends (or at least one end), and a gap is reserved between the filter mesh 332 and the filter cartridge 331 to provide a filtering function without affecting the flow velocity. The filter mesh 332 has a semi-enclosed structure with an opening at one end, and the opening of the filter mesh 332 is connected to the outlet end of the flow controller 32. The filter mesh 332 is made of a woven material with meshes. When blood flows from the flow controller 32 into the filter mesh 332, thrombi are retained within the filter mesh 332, and the filtered blood exits from the filter mesh 332. Because the filter mesh 332 has the semi-enclosed structure with the opening at one end, blood can flow out from all directions of the filter mesh 332. Even if a portion of the filter mesh 332 is blocked by thrombi, other portions of the filter mesh 332 have a filtering function, thereby preventing blockage of the revascularization system during the surgery.

In another embodiment, the filter mesh 332 is directly embedded into the lumen and a gap is reserved between the filter mesh 332 and the inner wall of the lumen, thereby achieving a filtering function without affecting the flow velocity.

Since the revascularization system of this embodiment is used conjunction with carotid artery stent implantation surgery, thrombi may detach throughout the surgery, a circumference of a minimum cross-section of the filter mesh 332 is at least twice a circumference of a maximum cross-section of the flow channel 3211. This ensures that the filter mesh 332 has a sufficiently large filtering area, thereby preventing complete blockage of the filter mesh 332 by thrombi. Preferably, the circumference of the minimum cross-section of the filter mesh 332 is 2.5 times that of the main section 32113 of the flow channel 3211, thereby avoiding an excessively small gap between the filter mesh 332 and the filter cartridge 331 due to an excessively large circumference of the minimum cross-section of the filter mesh.

Based on this, as shown in FIG. 22, the flow channel 3211 further includes a flared section 32114 arranged behind the main section 32113. The inner diameter of the flared section 32114 gradually increases from the inlet end 3211a to the outlet end 3211b to transition between the flow channel 3211 and the filter mesh 332.

In other embodiments, the blood filter may include a plurality of filter meshes arranged at intervals in an axial direction. Mesh diameters of the filter meshes gradually increase in a direction from an inlet to an outlet of the blood filter, thereby enabling multi-layer blood filtration and enhancing the filtration effect of the revascularization system.

The one-way check valve 34 may be of any suitable type commonly used in medical devices, and allows fluid to flow in only one direction. Suitable examples of check valves include, but are not limited to, seal ring valves, protector valves, ball check valves, diaphragm check valves, swing check valves, butterfly check valves, lift check valves, duckbill valves, and the like. It should be noted that in practical applications, if the revascularization system is used to communicate an artery to a vein, where a pressure difference between the artery and vein is large, the natural pressure difference can help achieve the same one-way flow effect as the check valve 34. In such cases, the check valve may be omitted. However, when the revascularization system is used to communicate an artery to an artery or communicate a vein to a vein, where a pressure difference is relatively small, the check valve 34 is necessary.

Referring to FIG. 19 again, the blood filter includes a filtration outlet channel 3311. The filtration outlet channel 3311 is connected with the one-way check valve 34. The inner diameter of the filtration outlet channel 3311 is denoted as d, and an inner diameter of the blood filter is denoted as D, with the condition that d ≤ 1/3D. In this embodiment, d = 3/16D. When blood flows through the blood filter 33, due to the larger diameter of the blood filter 33, the blood flow velocity is significantly reduced. The one-way check valve is primarily opened by an impact force of the blood flow. By setting a smaller inner diameter for the filtration outlet channel 3311, the blood flow velocity can be increased, thereby generating a greater impact force on the one-way check valve and enabling the blood to flow through the one-way valve smoothly. At the same time, the higher flow velocity of the blood helps further prevent reverse blood flow.

The revascularization system has an insulation function. Specifically, in this embodiment, the inflow tube 2 and the outflow tube 4 are both covered with insulating materials, such as rubber-plastic insulation tubes or foam insulation tubes. In other embodiments, constant temperature heating apparatus may further be added to the inflow tube 2, the outflow tube 4, and the venous sheath 5 to maintain a constant blood temperature.

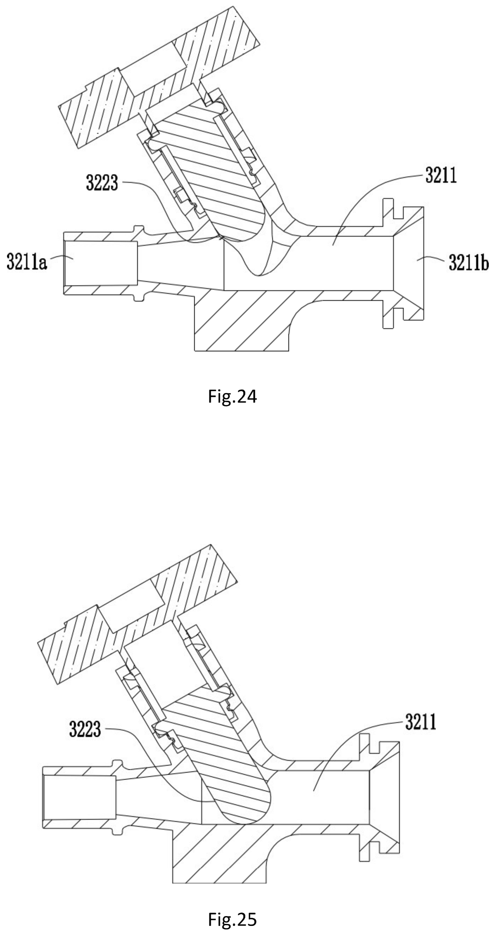

In another alternative embodiment, a flow controller 32 differs from the flow controller 32 in the previous embodiment mainly in the following way: as shown in FIG. 23 and FIG. 24, an included angle between a central axis of a flow channel 3211 and a central axis of a movable channel 3212 is an acute angle, the movable channel 3212 is inclined toward an inlet end of a main body element 321, and part of an outer peripheral surface of a flow control element 322 forms a guiding surface 3223. Specifically, the distal end of the flow control element 322 is in a cylinder-like shape, and when the distal end of the flow control element 322 extends into the flow channel 3211 and blocks the flow channel 3211, blood flows along the outer peripheral surface of the flow control element 322, and the outer peripheral surface of the flow control element 322 is inclined and curved relative to the blood flow direction, making it difficult for thrombi to accumulate and ensuring smoothness of the revascularization system.

Preferably, the flow control element 322 includes a knob 3221 and a sliding member 3222. The knob 3221 is rotatably arranged at a proximal end of the movable channel 3212, and an accommodating cavity is arranged at a distal end of the knob 3221. The sliding member 3222 is arranged in the accommodating cavity. The sliding member 3222 has a radially extending protrusion, the protrusion passes through a through slot in a side wall of the accommodating cavity and is in threaded connection with the movable channel 3212. When the knob 3221 is rotated under an external force, the protrusion presses against the through slot, and causes the sliding member 3222 to rotate together with the knob. Since the sliding member 3222 is in threaded connection with the movable channel 3212, the sliding member 3222 moves helically in the movable channel 3212 as the knob 3221 is rotated. Because the sliding member 3222, which is in direct contact with the blood, is accommodated in the accommodating cavity of the knob 3221, an operator can move the sliding member 3222 in the movable channel 3212 by rotating the knob 3221, thus preventing contamination of the blood within the movable channel 3212 by an external environment.

Preferably, the entire sliding member 3222 is in a cylinder-like shape and has a diameter that is the same as that of the flow channel 3211. A tail end of the sliding member 3222 is hemispherical and the diameter of the hemispherical tail end is the same as the inner diameter of the flow channel 3211. Thus, as shown in FIG. 25, the distal end of the sliding member can be closely fitted with the flow channel 3211, thereby allowing the flow controller 32 to achieve a hemostatic function. An outer peripheral surface of the sliding member 3222 forms the guiding surface. It should be noted that the outer peripheral surface includes a curved surface coaxial with the central axis of the sliding member 3222, as well as a curved surface at a distal end portion of the sliding member 3222.

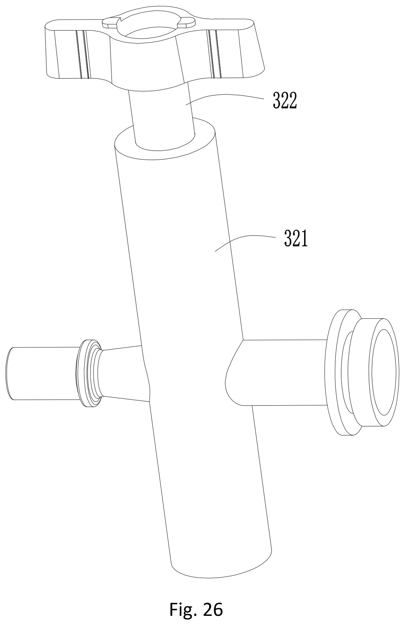

In an alternative embodiment, as shown in FIG. 26 to FIG 28, the flow control element 322 is provided with a plurality of communication holes 3224, and the extension direction of the communication holes 3224 is the same as the extension direction of the flow channel 3211. At least one of the communication holes 3224 includes a velocity adjustment section 32241. The entire velocity adjustment section 32241 has a funnel-shaped structure that gradually narrows in a direction from an inlet end 3211a to an outlet end 3211b. The guiding surface 3223 is formed on an inner wall of the velocity adjustment section 32241. An edge of the inlet end of the velocity adjustment section 32241 is in smooth transition connection with an inner wall of the flow channel 3211.

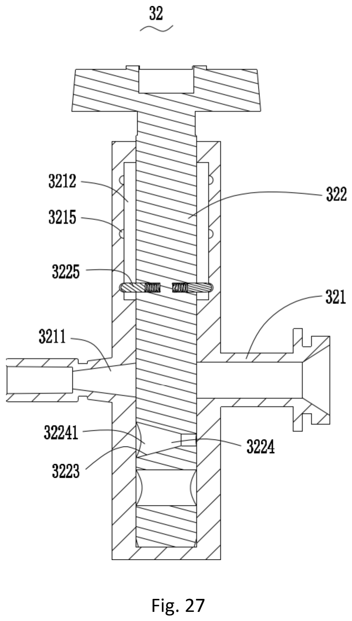

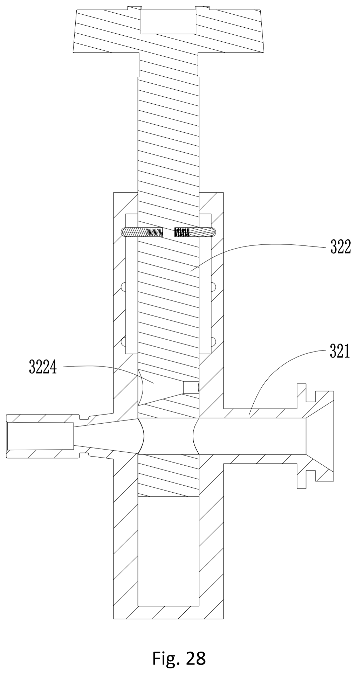

The flow control element 322 is provided with a plurality of communication holes 3224 with different diameters, as shown in FIG. 27 and FIG. 28. Under an external force, the flow control element 322 moves along the movable channel 3212, blood flows through the communication holes 3224 with different diameters, and then the flow rate of blood is regulated to a different extent. When none of the communication holes are aligned with the flow channel, the flow channel is blocked, and the flow controller is in a closed state. Additionally, since the edge of the inlet end of the velocity adjustment section 32241 is in smooth transition connection with the inner wall of the flow channel 3211, thrombi are less likely to accumulate at the flow control element 322.

The main body member 321 is provided with a plurality of first fitting portions 3215, and the flow control element 322 is provided with a plurality of second fitting portions 3225. When any of the first fitting portions 3215 is matched with any of second fitting portions 3225, correspondingly, the edge of the inlet end of the velocity adjustment section 32241 is in smooth transition connection with the inner wall of the flow channel 3211. In this embodiment, the first fitting portion 3215 is an arcuate hole in the inner wall of the flow channel 3211, and the first fitting portion 3215 is a spring block that can radially extend or contract along the flow control element 322. The tail end of the spring block is arcuate, and when the sliding block is inserted into the arcuate hole, the communication hole 3224 is aligned with the flow channel 3211, and correspondingly, the edge of the inlet end of the velocity adjustment section 32241 is in smooth transition connection with the inner wall of the flow channel 3211. When the flow control element 322 moves along the movable channel 3212 under an external force, the spring block is compressed back into the flow control element 322 until the spring block reaches the next arcuate hole. This structure enables an operator to precisely operate the flow control element 322, and ensures that the communication hole 3224 is aligned with the flow channel 3211 during the procedure.

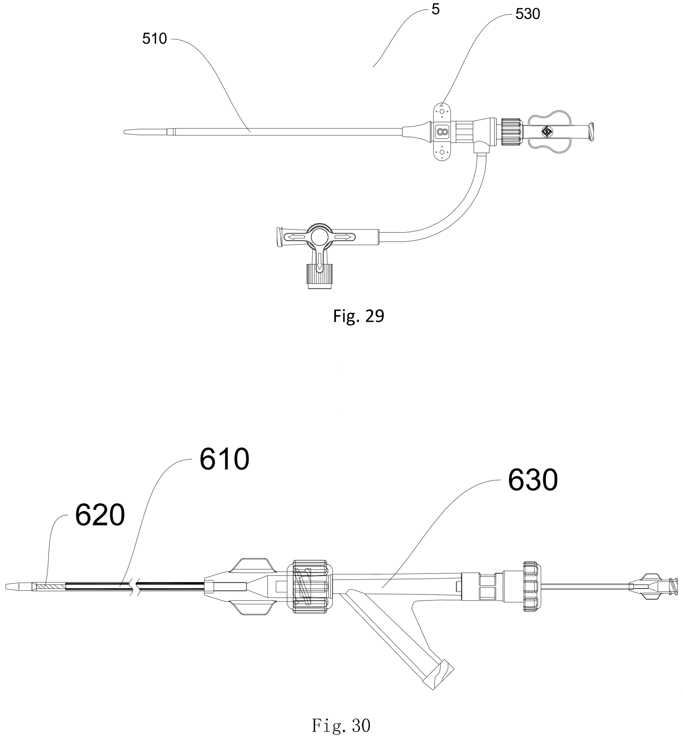

Referring to FIG. 29, in this embodiment, since the venous sheath 5 is used to transfer blood from the artery to the vein, the venous sheath 5 includes a sheath main body 510, and there is no need for a balloon structure on a side of the sheath main body 510 that is close to the vein.

In addition, the venous sheath 5 is provided with a stress tube 530 with suture holes, thereby preventing the sheath from bending and facilitating the fixation of the sheath.

In this embodiment, a proximal end of the sheath hub 1200 is connected to a stent system 6, which is used for implanting a stent in the carotid artery. It should be noted that, with respect to the stent, the revascularization system also involves a plurality of implementations as follows: referring to FIG. 30, the stent system 6 includes a stent sheath 610, and a distal end of the stent sheath 610 is provided with a stent loading area 620. The stent sheath 610 is connected to a handle 630.

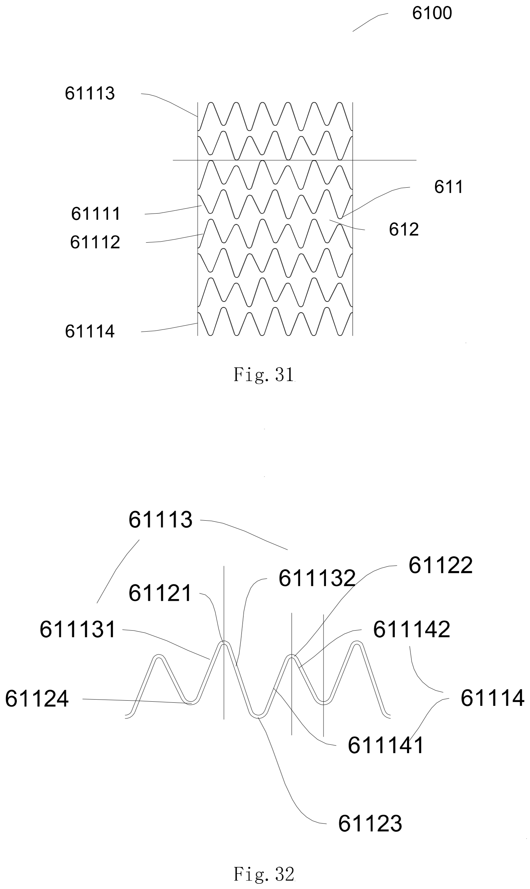

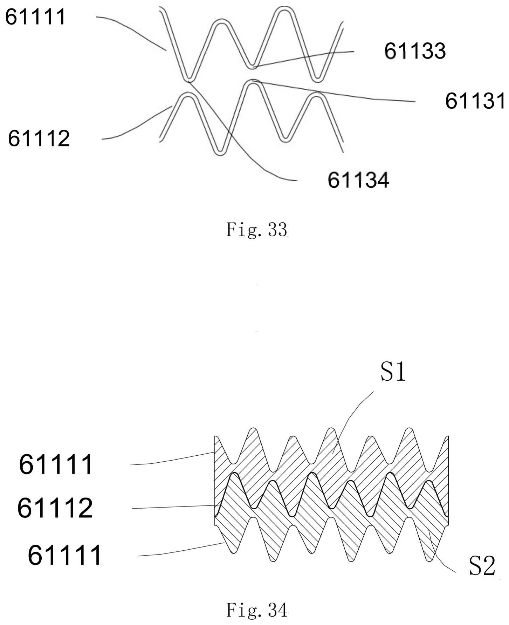

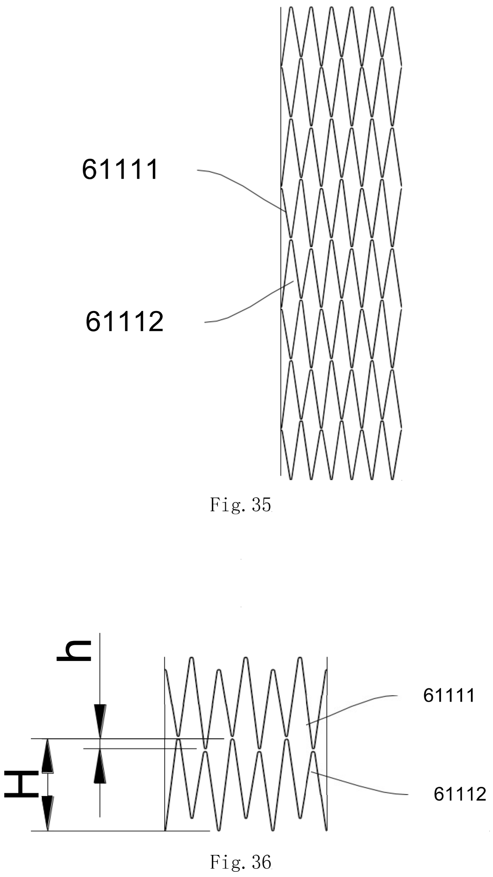

Referring to FIG. 31 to FIG. 36, FIG. 31 is a schematic structural diagram of a stent 6100 in a first embodiment of the present disclosure; FIG. 32 is a schematic structural diagram of a first corrugated ring 61111 of the stent 6100 in the first embodiment; FIG. 33 is a schematic diagram of positions of the first corrugated ring 61111 and a second corrugated ring 61112 of the stent 6100 in the first embodiment; FIG. 34 is a schematic diagram of an enclosed area of the first corrugated ring 61111 and the second corrugated ring 61112 of the stent 6100 in the first embodiment; FIG. 35 is a schematic diagram of an overall state of the stent 6100 in a compressed state in the first embodiment; and FIG. 36 is a schematic diagram of a partial position of the stent 6100 in the compressed state in the first embodiment of the present disclosure.

In this embodiment, the stent 6100 includes a stent main body 611, and a covering film 612 that covers a surface of the stent main body 611. The stent main body 611 includes a plurality of corrugated annular elements arranged axially along a central line of the stent. The corrugated annular elements are typically made of materials with good biocompatibility, such as nitinol, stainless steel, and cobalt-chromium alloys. The covering film 612 is made from a biocompatible polymer material, such as expanded polytetrafluoroethylene (ePTFE), polyethylene terephthalate (PET), or polyester fabric.

The stent main body 611 includes a plurality of corrugated rings 6111, which may take various shapes, such as Z-shaped, U-shaped, or sinusoidal waves, and may be freely adjusted as needed.

In this embodiment, the corrugated rings 6111 include a plurality of first corrugated rings 61111, a plurality of second corrugated rings 61112, which are sequentially arranged, and a proximal corrugated ring 61113 and a distal corrugated ring 61114 which are respectively located at the proximal end and the distal end. The first corrugated rings 61111 and the second corrugated rings 61112 are adjacent corrugated rings in the plurality of corrugated rings 6111.

In this embodiment, crests of waveforms of the proximal corrugated ring 61113 are at the same height, i.e., the proximal side of the proximal corrugated ring 61113 is flat. Similarly, troughs of waveforms of the distal corrugated ring 61114 are aligned, i.e., the distal side of the distal corrugated ring 61114 is flat. This ensures that the corrugated rings at the end portions remain flat after compression and release, thereby maintaining the stability of two end portions of the stent main body 611. Additionally, it ensures that the proximal corrugated ring 61113 provides uniform support to the covering film 612 on the proximal side, while the distal corrugated ring 61114 provides uniform support to the covering film 612 on the distal side.

In this embodiment, each first corrugated ring 61111 includes a first wave 61113 and a second wave 61114 which are in continuous distribution, where the first wave 61113 is a high wave and the second wave 61114 is a low wave. The first wave 61113 includes a first wave strut 611131 and a second wave strut 611132, while the second wave 61114 includes a third wave strut 611141 and a fourth wave strut 611142. These struts satisfy the following relationships.

For the entire first corrugated ring 61111, the first wave strut 611131 is adjacent to the second wave strut 611132 and the fourth wave strut 611142; the second wave strut 611132 is adjacent to the first wave strut 611131 and the third wave strut 611141; and the third wave strut 611141 is adjacent to the second wave strut 611132 and the fourth wave strut 611142, and smooth transition between the adjacent wave struts is achieved.

Therefore, in this embodiment, each first corrugated ring 61111 includes at least two crests: a high crest 61121 (between the first wave strut 611131 and the second wave strut 611132) and a low crest 61122 (between the third wave strut 611141 and the fourth wave strut 611142). The first corrugated ring 61111 further includes two troughs: a high trough 61123 (between the first wave strut 611131 and the adjacent fourth wave strut 611142) and a low trough 61124 (between the second wave strut 611132 and the third wave strut 611141).

In this embodiment, the second corrugated ring 61112 is selected to have the same waveform structure as the first corrugated ring 61111. However, a phase difference is provided between the second corrugated ring 61112 and the first corrugated ring 61111, such that the low trough 61124 of the first corrugated ring 61111 corresponds to a low crest 61122 of the second corrugated ring 61112, and the high trough 61123 corresponds to a high crest 61113 of the second corrugated ring 61112. As a result, in this design, when the stent main body 611 of this embodiment is compressed, the adjacent first corrugated ring 61111 and second corrugated ring 61112 do not axially overlap. In other words, they do not overlap by axially compressing the covering film 612, but rather, the crests and the troughs abut against each other to achieve a limiting effect. This design prevents excessive shortening of the stent, thereby reducing the risk of jumping before the stent is deployed. When the stent expands, the corrugated rings are staggered, thereby preventing partial collapse due to excessively large pores in the covering film 612, ensuring that the corrugated rings do not interfere with each other, and maintaining the flexibility of the stent.

In this embodiment, an insertion height h between the first corrugated ring 61111 and the second corrugated ring 61112 and a height H of a first wave 61113 satisfies h<H, which prevents torsional damage to a vessel wall during stent deployment.

Further, to ensure that the covering film 612 is uniformly compressed between the first corrugated ring and the second corrugated ring during compression of the stent 6100, and that there is no abnormal increase in an axial sectional area of the covering film inside a delivery device due to compression, an area S1 of the covering film 612 between the first corrugated ring 61111 and the second corrugated ring 61112 is equal to an area S2 of the covering film 612 between the adjacent first corrugated ring 61111 and second corrugated ring 61112. This ensures that when the plurality of first corrugated rings 61111 and the plurality of second corrugated rings 61112 are compressed, the deformation of the covering films 612 on both an upper side and a lower side is nearly identical.

In this embodiment, the plurality of low troughs 61124 of the first corrugated ring 61111 and the plurality of high crests 61121 of the adjacent second corrugated ring 61112 are located at the same height (i.e., at the same axial level). This design avoids the situation where the covering film 612 between the first corrugated ring 61111 and the second corrugated ring 61112 lacks axial support from the corrugated rings, thus preventing axial shortening of the stent.

In another embodiment, referring to FIG. 32, the axial position of the high crest 61131 of the second corrugated ring 61112 is located between axial positions of a high trough 61133 and a low trough 61134 of the first corrugated ring 61111. As a result, when the stent undergoes torsion, the high crest 61131 is constrained by the high trough 61133 and low trough 61134, thereby preventing excessive torsion of the stent.

It should be noted that in another embodiment, the stent 6100 is a bare stent.

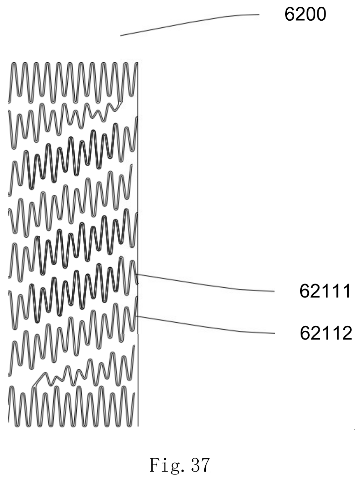

In another alternative embodiment, the first corrugated ring and the second corrugated ring are arranged in spiral distribution. Referring to FIG. 37, a first corrugated ring 62111 and a second corrugated ring 62112 are in spiral distribution.

To ensure that a covering film 622 is uniformly compressed between the first corrugated ring and the second corrugated ring during compression of a vascular stent 6200 of this embodiment, and that there is no abnormal increase in an axial sectional area of the covering film inside a delivery device due to compression, in this embodiment, the area of the covering film 622 between the first corrugated ring 62111 and the second corrugated ring 62112 is equal to the area of the covering film 622 between the adjacent first corrugated ring 62111 and second corrugated ring 62112. This ensures that when the plurality of first corrugated rings 62111 and the plurality of second corrugated rings 62112 are compressed, the deformation of the covering films 622 on both an upper side and a lower side is nearly identical. However, unlike the previous embodiment, in this embodiment, the area S1 of the covering film 622 between the first corrugated ring 62111 and the second corrugated ring 62112, as well as the area S2 between the adjacent first corrugated ring 62111 and second corrugated ring 62112, refer to a complete circumferential coverage of the first corrugated ring 62111 and the second corrugated ring 62112.

In another embodiment, the first corrugated ring and the second corrugated ring are continuous, that is, the first corrugated ring and the second corrugated ring are continuous corrugated rings which are arranged along the same helical pattern.

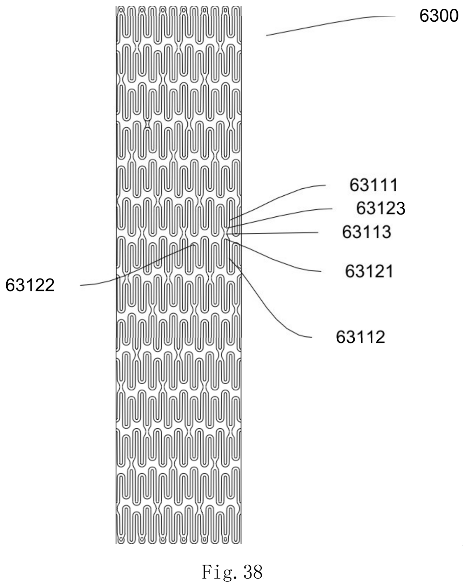

In another alternative embodiment, the first corrugated ring and the second corrugated ring are connected with each other. Referring to FIG. 38, which is a schematic structural diagram of a vascular stent according to another embodiment of the present disclosure, a first corrugated ring 63111 and a second corrugated ring 63112 of this embodiment include a plurality of connection points 63113. Specifically, the connection points 63113 are located between high troughs 63123 of the first corrugated ring 63111 and high crests 63121 of the second corrugated ring 63112. Additionally, the two adjacent connection points 63113 are separated by two low crests 63122 of the second corrugated ring 63112.

To ensure that a covering film 632 is uniformly compressed between the first corrugated ring and the second corrugated ring during compression of a vascular stent 6300 of this embodiment, and that there is no abnormal increase in an axial sectional area of the covering film inside a delivery device due to compression, in this embodiment, the area of the covering film 632 between the first corrugated ring 63111 and the second corrugated ring 63112 is equal to the area of the covering film 632 between the adjacent first corrugated ring 63111 and second corrugated ring 63112. This ensures that when the plurality of first corrugated rings 63111 and the plurality of second corrugated rings 63112 are compressed, the deformation of the covering films 632 on both an upper side and a lower side is nearly identical. However, unlike the previous embodiment, this embodiment also ensures that the deformation of the covering film 632 on both sides of the connection point 63113 is nearly identical. In other words, the area S enclosed by two adjacent connection points 63113 and the first corrugated ring 63111 and the second corrugated ring 63112 is uniform.

In this embodiment, the overall area of meshes may be adjusted by adjusting the number of connection points 63113. Generally, more connection points 63113 result in more areas enclosed by two adjacent connection points 63113 and the first corrugated ring 63111 and the second corrugated ring 63112, thereby forming a closed-loop structure and resulting low flexibility. Conversely, fewer connection points 63113 result in fewer areas enclosed by two adjacent connection points 63113 and the first corrugated ring 63111 and the second corrugated ring 63112, thereby forming an open-loop structure and resulting in higher flexibility.

In another embodiment, the area between two adjacent connection points 63113 includes a plurality of low crests 63122.

In another embodiment, a relatively small number of connection points are arranged at two end portions of the vascular stent, such that the end portions are close to or form an open-loop structure. This configuration results in larger covering film areas, prevents the detachment of stenotic plaques, avoids excessively poor flexibility at the end portions of the vascular stent, and ensures adequate conformability and adherence to the vessel walls.

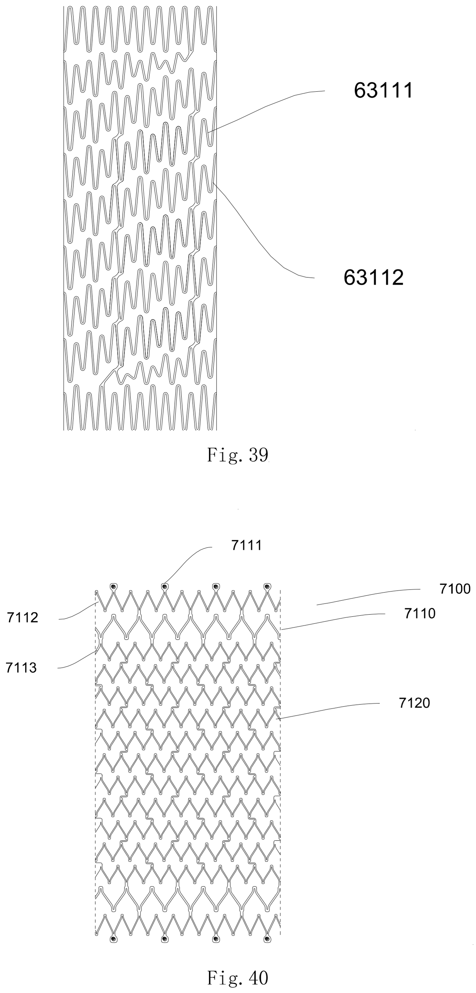

In another embodiment, referring to FIG. 39, which is a schematic structural diagram of a vascular stent according to another embodiment of the present disclosure, both first corrugated rings 63111 and second corrugated rings 63112 are in inclined distribution.

Overall, the revascularization system of this embodiment has a relatively short overall length, which reduces the risk of excessive release force or positional deviation caused by a long torque transmission distance and minimizes the risk of damage to vessel walls in a delivery path during long-distance manipulation. Furthermore, the revascularization system of this embodiment belongs to a proximal cerebral protection system, and has advantages over a distal cerebral protection system. Specifically, the distal cerebral protection system is a standard approach used in carotid artery stenting (CAS), where a guide wire first extends through a stenotic area, and then an umbrella-type protection device is delivered along the guide wire; the distal cerebral protection system does not alter the direction of blood flow; and when devices such as balloons or stents are used in the stenotic area, detached plaques follow antegrade blood flow toward the umbrella-type protection device, and after the stent is deployed, the umbrella-type protection device is retrieved. In contrast, the proximal cerebral protection is achieved by occluding a proximal blood flow to induce reverse blood flow; when devices such as balloons and stents are deployed in the stenotic area, detached plaques are carried out of the body by the reverse blood flow; since there is no need to retrieve an umbrella-type protection device, there is no risk of plaque escape during umbrella-type protection device retrieval; and moreover, the proximal cerebral protection system avoids the problem of dislodging plaques when passing through the stenotic area, which is common with the distal cerebral protection system, and eliminates the risk of plaque escape from the umbrella-type protection device due to poor retraction performance or oversized meshes during the retrieval phase.

In another embodiment, refer to FIG. 40. FIG. 40 is a schematic structural diagram of a vascular stent according to another embodiment of the present disclosure, illustrating an expanded configuration of a stent 7100 as an alternative configuration. The stent 7100 substantially adopts an equal-amplitude wave structure, radiopaque sections 7110 are arranged at both a proximal end and a distal end, and a main section 7120 is arranged in a middle area. Specifically, each radiopaque section 7110 includes a plurality of radiopaque markers 7111 which are located at an end portion and distributed in a circumferential direction. An outer ring 7112 of the radiopaque section 7110 is located at an edge of the end portion, while an inner ring 7113 is on a side close to the main section 7120. A plurality of intermediate rings may also be arranged between the outer ring 7112 and the inner ring 7113 of the radiopaque section 7110.

In another embodiment, as an alternative solution suitable for applications where minimizing stent dimensions is required, the radiopaque section 7110 may be arranged on only one side of the main section 7120, that is, the radiopaque section 7110 may be arranged on either a proximal side or distal side of the main section 7120. This facilitates the independent determination of the position of either the proximal side or distal side of the main section 7120.

It should be noted that in the radiopaque section 7110, a connection section between adjacent corrugated rings extends in an axial direction. This design ensures the axial integrity of the radiopaque section 7110 and prevents overlapping of the radiopaque section 7110 from compromising radiopaque effect.

In the main section 7120, a connection section between adjacent corrugated rings substantially extends in a circumferential direction. Adjacent corrugated rings are offset by one-quarter of a wavelength in the circumferential direction, such that the crests and troughs of adjacent rings are non-collinear. Because the connection section between adjacent corrugated rings extends substantially in the circumferential direction (in a "∫" shape), the main section 7120 is less prone to circumferential overlapping during compression or expansion, i.e., the main section is less prone to rotation. This helps avoid internal stress concentration on a single side, which could otherwise reduce the service life of the stent or hinder its smooth advancement into or withdrawal from a sheath.

In another embodiment, the radiopaque markers 7111 are located at crests of an edge corrugated ring, and connection points 7112 between the edge corrugated ring and an intermediate corrugated ring are located at circumferential midpoints between two adjacent radiopaque markers.

Each technical feature of the above embodiments may be freely combined. For brief description, not all possible combinations of each technical feature in the abovementioned embodiments are described, but all the combinations of these technical features shall fall within the scope recorded in the description without conflicts.

The abovementioned embodiments only express some implementation modes of the present disclosure and are specifically described in detail and not thus understood as limits to the patent scope of the present disclosure. It is to be pointed out that those of ordinary skill in the art may further make a plurality of transformations and improvements without departing from the concept of the present disclosure and all of these fall within the scope of protection of the present disclosure. Therefore, the scope of patent protection of the disclosure should be subject to the appended claims.

## Claims

1. A revascularization system, comprising a first sheath, a bypass system, and a second sheath, which are sequentially connected, wherein
the first sheath comprises a main body and a catheter seat located at a proximal end of the main body; a proximal end of the catheter seat is communicated with a stent system, and the stent system is configured to load and deliver a stent to a distal end of the main body; and
the bypass system comprises an inflow tube in communication with the first sheath, an outflow tube in communication with the second sheath, and a bypass assembly connected between the inflow tube and the outflow tube, the bypass assembly comprises a tube cavity in which a blood filter is arranged, and blood flows from the first sheath to the second sheath via the bypass system.

2. The revascularization system according to claim 1, wherein the first sheath comprises a tubular main body and a balloon located at a distal end of the main body, the balloon is located on a distal side of the first sheath, and an inflation port of the balloon is communicated with the balloon via the catheter seat.

3. The revascularization system according to claim 2, wherein the main body comprises an inflation lumen which is communicated with the balloon and the catheter seat, and when inflated, the balloon blocks an outer periphery of the main body and exposes an inner lumen of the main body.

4. The revascularization system according to claim 3, wherein the main body further comprises at least one angiography lumen.

5. The revascularization system according to claim 1, wherein the bypass system comprises a flow controller connected to an end of the inflow tube that is away from the first sheath; the flow controller comprises a main body element with a flow channel and a flow control element, and the flow control element is at least partially accommodated in the main body element and movably connected to the flow channel; and under an external force, the flow control element reduces or increases a flow area of the flow channel.

6. The revascularization system according to claim 1, wherein the bypass system comprises a one-way check valve, which is connected between the blood filter and the outflow tube, or connected to the outflow tube.

7. The revascularization system according to claim 1, wherein the flow channel is provided with an inlet end and an outlet end; the inlet end is close to the inflow tube and the outlet end is far away from the inflow tube; the flow control element is provided with a guiding surface oriented to the inlet end; and the guiding surface and an inner surface of the flow channel define a flow-regulating channel that tapers from the inlet end to the outlet end.

8. The revascularization system according to claim 4, wherein the blood filter comprises a filter mesh that is spaced from an inner wall of the tube cavity; or the blood filter further comprises a filter cartridge which has a tubular structure with at least one open end; and the filter mesh is arranged inside the filter cartridge and is spaced from an inner wall of the filter cartridge.

9. The revascularization system according to claim 1, wherein the flow channel comprises a connection section, a constricted section, and a main section, which are sequentially connected from the inlet end to the outlet end; and an inner diameter of the constricted section is smaller than that of the connection section and the main section.

10. The revascularization system according to claim 2, wherein the catheter seat comprises at least a first cavity, a second cavity, and a third cavity; the first cavity and the third cavity are located on two sides of the second cavity respectively; a first connection plate is connected with the first cavity and the second cavity; a second connection plate is connected with the third cavity and the second cavity; and each of the first connection plate and the second connection plate is provided with at least one suture hole.

11. The revascularization system according to claim 1, wherein the stent comprises a plurality of main corrugated rings; each corrugated ring comprises at least two crests and/or troughs of different heights; and the main corrugated rings comprise a first corrugated ring and a second corrugated ring, between which a phase difference is provided, such that a plurality of troughs of the first corrugated ring are aligned with a plurality of crests of the second corrugated ring, and/or a plurality of crests of the first corrugated ring are aligned with a plurality of troughs of the second corrugated ring.

12. The revascularization system according to claim 11, wherein the first corrugated ring and the second corrugated ring are in an inclined distribution.

13. The revascularization system according to claim 1, wherein the stent comprises a main section and a radiopaque section located on at least one side of the main section, and the radiopaque section comprises a plurality of radiopaque markers distributed in a circumferential direction.

14. The revascularization system according to claim 13, wherein in the radiopaque section, a connection section between adjacent corrugated rings extends in an axial direction.

15. The revascularization system according to claim 13, wherein in the main section, a connection section between adjacent corrugated rings extends substantially in the circumferential direction
